(19) Europäisches Patentamt
European Patent Office
Office européen des brevets

(11) **EP 2 726 020 B1**

(12) **EUROPEAN PATENT SPECIFICATION**

(45) Date of publication and mention of the grant of the patent:
**04.11.2015 Bulletin 2015/45**

(21) Application number: **12803639.9**

(22) Date of filing: **27.06.2012**

(51) Int Cl.:
***A61F 2/38*** *(2006.01)*

(86) International application number:
**PCT/US2012/044354**

(87) International publication number:
**WO 2013/003433 (03.01.2013 Gazette 2013/01)**

(54) **POSTERIOR STABILIZED ORTHOPAEDIC KNEE PROSTHESIS HAVING CONTROLLED CONDYLAR CURVATURE**

ORTHOPÄDISCHE KNIEPROTHESE MIT VORDERSTABILISIERUNG UND GESTEUERTER KONDYLUSKRÜMMUNG

PROTHÈSE DE GENOU ORTHOPÉDIQUE STABILISÉE POSTÉRIEURE AVEC COURBURE CONDYLIENNE COMMANDÉE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.06.2011 US 201161503343 P**

(43) Date of publication of application:
**07.05.2014 Bulletin 2014/19**

(73) Proprietor: **DePuy (Ireland)**
**Ringaskiddy Cork (IE)**

(72) Inventors:
• **WYSS, Joseph G.**
**Fort Wayne, Indiana 46814 (US)**

• **LEE, Jordan S.**
**Warsaw, Indiana 46582 (US)**
• **WAGNER, Christel M.**
**Plymouth, Indiana 46563 (US)**

(74) Representative: **Belcher, Simon James**
**Urquhart-Dykes & Lord LLP**
**Tower North Central**
**Merrion Way**
**Leeds LS2 8PA (GB)**

(56) References cited:
| | |
|---|---|
| **WO-A1-96/23460** | **US-A- 3 765 033** |
| **US-A1- 2009 326 665** | **US-A1- 2009 326 665** |
| **US-A1- 2009 326 666** | **US-B1- 7 081 137** |

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

The invention relates to orthopaedic prostheses for use in knee replacement surgery.

Joint arthroplasty is a well-known surgical procedure by which a diseased and/or damaged natural joint is replaced by a prosthetic joint. A typical knee prosthesis includes a tibial tray, a femoral component, and a polymer insert or bearing positioned between the tibial tray and the femoral component. Depending on the severity of the damage to the patient's joint, orthopaedic prostheses of varying mobility may be used. For example, the knee prosthesis may include a "fixed" tibial bearing in cases in which it is desirable to limit the movement of the knee prosthesis, such as when significant soft tissue damage or loss is present. Alternatively, the knee prosthesis may include a "mobile" tibial bearing in cases in which a greater degree of freedom of movement is desired. Additionally, the knee prosthesis may be a total knee prosthesis designed to replace the femoral-tibial interface of both condyles of the patient's femur or a uni-compartmental (or uni-condylar) knee prosthesis designed to replace the femoral-tibial interface of a single condyle of the patient's femur.

The type of orthopaedic knee prosthesis used to replace a patient's natural knee may also depend on whether the patient's posterior cruciate ligament is retained or sacrificed (i.e., removed) during surgery. For example, if the patient's posterior cruciate ligament is damaged, diseased, and/or otherwise removed during surgery, a posterior stabilized knee prosthesis may be used to provide additional support and/or control at later degrees of flexion. Alternatively, if the posterior cruciate ligament is intact, a cruciate retaining knee prosthesis may be used.

Typical orthopaedic knee prostheses are generally designed to duplicate the natural movement of the patient's joint. As the knee is flexed and extended, the femoral and tibial components articulate and undergo combinations of relative anterior-posterior motion and relative internal-external rotation. However, the patient's surrounding soft tissue also impacts the kinematics and stability of the orthopaedic knee prosthesis throughout the joint's range of motion. That is, forces exerted on the orthopaedic components by the patient's soft tissue may cause unwanted or undesirable motion of the orthopaedic knee prosthesis. For example, the orthopaedic knee prosthesis may exhibit an amount of unnatural (paradoxical) anterior translation as the femoral component is moved through the range of flexion.

In a typical orthopaedic knee prosthesis, paradoxical anterior translation may occur at nearly any degree of flexion, but particularly at mid to late degrees of flexion. Paradoxical anterior translation can be generally defined as an abnormal relative movement of a femoral component on a tibial bearing in which the contact "point" between the femoral component and the tibial bearing "slides" anteriorly with respect to the tibial bearing. This paradoxical anterior translation may result in loss of joint stability, accelerated wear, abnormal knee kinematics, and/or cause the patient to experience a sensation of instability during some activities.

US-A-2009/0326665 discloses a posterior stabilised orthopaedic knee prosthesis having the features specified in the pre-characterising part of claim 1.

The invention provides a posterior stabilized orthopaedic knee prosthesis as defined in claim 1.

Optionally, the femoral component may initially contact the spine of the tibial bearing at a degree of flexion in the range of 70 degrees to 80 degrees.

Optionally, the first degree of flexion is about 0 degrees and the second degree of flexion is about 70 degrees.

Optionally, the third degree of flexion is no less than 73 degrees.

Optionally, the first radius of curvature may be greater than the second radius of curvature and the first curved surface section may have an anterior-posterior decreasing, non-constant radius of curvature.

Optionally, the condyle surface of the femoral component may also contact the bearing surface at a fourth contact point on the condyle surface at a fourth degree of flexion. The fourth degree of flexion may be greater than the third degree of flexion. Optionally, the fourth degree of flexion is in the range of 90 degrees to 120 degrees. The condyle surface may also include a fourth radius of curvature in the sagittal plane at the fourth contact point. The ratio of the fourth radius of curvature to the third radius of curvature may then be in the range of 0.7 to 1.15.

Optionally, the condyle surface of the femoral component may also contact the bearing surface at a fifth contact point on the condyle surface at a fifth degree of flexion. The fifth degree of flexion may be greater than the fourth degree of flexion. Optionally, the fifth degree of flexion is in the range of 140 degrees to 165 degrees. The condyle surface may also include a fifth radius of curvature in the sagittal plane at the fifth contact point. The fifth radius of curvature may then be less than the fourth radius of curvature.

In another posterior stabilized orthopaedic knee prosthesis, the condyle surface of the femoral component may contact the bearing surface at a first contact point on the condyle surface at a first degree of flexion, contact the bearing surface at a second contact point on the condyle surface at a second degree of flexion, and contact the bearing surface at a third contact point on the condyle surface at a third degree of flexion. Additionally, the posterior cam of the femoral component may initially contact the spine of the tibial bearing at a degree of flexion between the second degree of flexion and the third degree of flexion. For example, the femoral component may initially contact the spine of the tibial bearing at a degree of flexion in the range of 70 degrees to 80 degrees.

The first degree of flexion may be about 0 degrees. The second degree of flexion may be greater than the first degree of flexion and may be in the range of about 60 degrees to about 75 degrees in some constructions. For example, the second degree of flexion is about 70 degrees. The third degree of flexion may be greater than the second degree and less than about 90 degrees. For example, the third degree of flexion is no less than 73 degrees.

The condyle surface of the femoral component may also contact the bearing surface at a plurality of contact points between the first contact point and the second contact point when the femoral component is moved from the first degree of flexion to the second degree of flexion. Optionally, each contact point of the plurality of contact points may be defined by a ray extending from a common origin to the respective contact point of the plurality of contact points, each ray having a length defined by the following polynomial equation:

$$r_\theta = (a + (b * \theta) + (c * \theta^2) + (d * \theta^3)),$$

in which $r_\theta$ is the length of the ray defining a contact point at $\theta$ degrees of flexion, a , b , c, and d are coefficient values. For example, in one construction, a is a coefficient value between 35 and 45, and b is a coefficient value in a range selected from the group consisting of: 0.00 < b < 0.30 and b = 0.015384615, in which when b is in the range of 0 < b < 0.30, (i) c is a coefficient value between -0.010 and 0.00 and (ii) d is a coefficient value between -0.00015 and 0.00, and in which when b is equal to 0.015384615, (i) c is a coefficient value equal to about -0.00027024 and (ii) d is a coefficient value equal to about -0.0000212.

Optionally, the condyle surface may have a first radius of curvature in the sagittal plane at the first contact point. The first radius of curvature may have an origin and the distance between the origin of the first radius of curvature and the common origin of the rays may be in the range of 0 and 10 mm. Additionally, the first radius of curvature may be greater than the second radius of curvature and the first curved surface section may have an anterior-posterior decreasing, non-constant radius of curvature.

In another posterior stabilized orthopaedic knee prosthesis, the condyle surface of the femoral component may contact the bearing surface at a first contact point on the condyle surface at a first degree of flexion, contact the bearing surface at a second contact point on the condyle surface at a second degree of flexion, and contact the bearing surface at a third contact point on the condyle surface at a third degree of flexion. Additionally, the posterior cam of the femoral component may initially contact the spine of the tibial bearing at a degree of flexion between the second degree of flexion and the third degree of flexion. For example, the femoral component may initially contact the spine of the tibial bearing at a degree of flexion in the range of 70 degrees to 80 degrees.

The second degree of flexion may be greater than the first degree of flexion and may be in the range of about 60 degrees to about 75 degrees in some constructions. For example, the second degree of flexion is about 70 degrees. The third degree of flexion may be greater than the second degree and less than about 90 degrees. For example, the third degree of flexion is no less than 73 degrees.

The condyle surface in the sagittal plane may have a first radius of curvature at the first contact point, a second radius of curvature at the second contact point, and a third radius of curvature at the third contact point. Additionally, the condyle surface may have a first curved surface section defined between the first contact point and the second contact point. The first curved surface section may have a decreasing, non-constant radius of curvature. The condyle surface may also have a second curved surface section defined between the second contact point and the third contact point. The second curved surface section may have a substantially constant radius of curvature equal to the third radius of curvature.

Optionally, the condyle surface of the femoral component may also contact the bearing surface at a plurality of contact points between the first contact point and the second contact point when the femoral component is moved from the first degree of flexion to the second degree of flexion. Optionally, each contact point of the plurality of contact points may be defined by a ray extending from a common origin to the respective contact point of the plurality of contact points, each ray having a length defined by the following polynomial equation:

$$r_\theta = (a + (b * \theta) + (c * \theta^2) + (d * \theta^3)),$$

in which $r_\theta$ is the length of the ray defining a contact point at $\theta$ degrees of flexion, a , b , c, and d are coefficient values. For example, a is a coefficient value between 35 and 45, and b is a coefficient value in a range selected from the group consisting of: 0.00 < b < 0.30 and b = 0.015384615, in which when b is in the range of 0 < b < 0.30, (i) c is a coefficient value between -0.010 and 0.00 and (ii) d is a coefficient value between -0.00015 and 0.00, and in which when b is equal to 0.015384615, (i) c is a coefficient value equal to about -0.00027024 and (ii) d is a coefficient value equal to about -0.0000212.

Optionally, each of the pair of spaced apart condyles may include a condyle surface. The condyle surfaces may then be substantially symmetrical or may be asymmetrical.

The detailed description particularly refers to the following figures, in which:

FIG. 1 is an exploded perspective view of an orthopaedic knee prosthesis.

FIG. 2 is a cross-sectional view of a femoral component and tibial bearing of FIG. 1 taken generally along section lines 2-2 and having the femoral component articulated to a first degree of flexion.

FIG. 3 is a cross-sectional view of a femoral component and tibial bearing of FIG. 2 having the femoral component articulated to a second degree of flexion.

FIG. 4 is a cross-sectional view of a femoral component and tibial bearing of FIG. 2 having the femoral component articulated to a third degree of flexion.

FIG. 5 is a cross-section view of the femoral component of FIG. 1.

FIG. 6 is a cross-section view of another femoral component of FIG. 1.

FIG. 7 is a cross-section view of another femoral component of FIG. 1.

FIG. 8 is a cross-section view of another femoral component of FIG. 1.

FIG. 9 is a graph of the anterior-posterior translation of a simulated femoral component having an increased radius of curvature located at various degrees of flexion.

FIG. 10 is a graph of the anterior-posterior translation of another simulated femoral component having an increased radius of curvature located at various degrees of flexion.

FIG. 11 is a graph of the anterior-posterior translation of another simulated femoral component having an increased radius of curvature located at various degrees of flexion.

FIG. 12 is a graph of the anterior-posterior translation of another simulated femoral component having an increased radius of curvature located at various degrees of flexion.

FIG. 13 is a cross-sectional view of another femoral component of FIG. 1.

FIG. 14 is a table containing a set of coefficient values of a polynomial equation defining the curve of the femoral component of FIG. 13 for a family of femoral component sizes.

FIG. 15 is a table containing a set of radii of curvature values and ratios for a family of femoral component sizes.

FIG. 16 is a cross-section view of another condyle of the femoral component of FIG. 1.

FIG. 17 is a perspective view of another femoral component of FIG. 1.

FIG. 18 is a cross-sectional view of the femoral component of FIG. 17.

FIG. 19 is a table containing a set of coefficient values of a polynomial equation defining a curve of the femoral component of FIGS. 17 and 18 for a family of femoral component sizes.

FIG. 20 is a table containing a set of radii of curvature values and ratios for a family of femoral component sizes of the femoral component of FIGS. 17 and 18.

FIG. 21 is a graph of the anterior-posterior translation of a simulated femoral component having an increased radius of curvature located at various degrees of flexion.

FIG. 22 is a cross-section view of another femoral component of FIGS. 17 and 18.

The description of devices which do not have all of the features of the invention is included in this document to aid understanding of the invention.

Terms representing anatomical references, such as anterior, posterior, medial, lateral, superior and inferior, may be used throughout this document to refer to both the orthopaedic implants described herein and a patient's natural anatomy. Such terms have well-understood meanings in both the study of anatomy and the field of orthopaedics. Use of such anatomical reference terms in this document is intended to be consistent with their well-understood meanings unless noted otherwise.

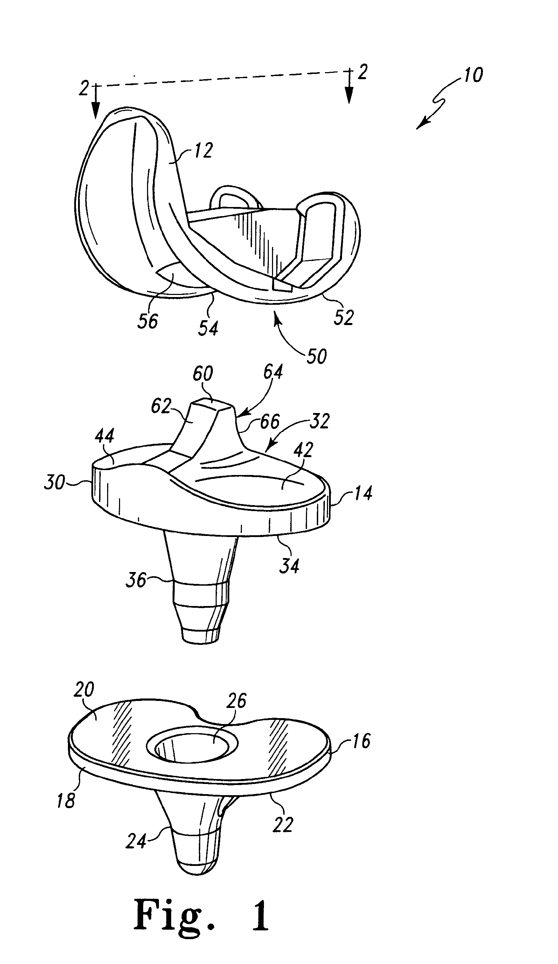

Referring now to FIG. 1, a posterior stabilized orthopaedic knee prosthesis 10 includes a femoral component 12, a tibial bearing 14, and a tibial tray 16. The femoral component 12 and the tibial tray 16 are illustratively formed from a metallic material such as cobalt-chromium or titanium, but may be formed from other materials, such as a ceramic material, a polymer material, a bio-engineered material, or the like. The tibial bearing 14 is illustratively formed from a polymer material such as a ultra-high molecular weight polyethylene (UHMWPE), but may be formed from other materials, such as a ceramic material, a metallic material, a bio-engineered material, or the like.

As discussed below, the femoral component 12 is configured to articulate with the tibial bearing 14, which is configured to be coupled with the tibial tray 16. The illustrative tibial bearing 14 is embodied as a rotating or mobile tibial bearing and is configured to rotate relative to the tibial tray 16 during use. However, the tibial bearing 14 may instead be embodied as a fixed tibial bearing, which may be limited or restricted from rotating relative the tibial tray 16.

The tibial tray 16 is configured to be secured to a surgically-prepared proximal end of a patient's tibia (not shown). The tibial tray 16 may be secured to the patient's tibia via use of bone adhesive or other attachment means. The tibial tray 16 includes a platform 18 having an top surface 20 and a bottom surface 22. Illustratively, the top surface 20 is generally planar and, may be highly polished. The tibial tray 16 also includes a stem 24 extending downwardly from the bottom surface 22 of the platform 18. A cavity or bore 26 is defined in the top surface 20 of the platform 18 and extends downwardly into the stem 24. The bore 26 is formed to receive a complimentary stem of the tibial insert 14 as discussed in more detail below.

As discussed above, the tibial bearing 14 is configured to be coupled with the tibial tray 16. The tibial bearing 14 includes a platform 30 having an upper bearing surface 32 and a bottom surface 34. In the construction in which the tibial bearing 14 is embodied as a rotating or mobile tibial bearing, the bearing 14 includes a stem 36 extending downwardly from the bottom surface 34 of the platform 30. When the tibial bearing 14 is coupled to the tibial tray 16, the stem 36 is received in the bore 26 of the tibial tray 16. In use, the tibial bearing 14 is configured to rotate about an axis defined by the stem 36 relative to the tibial tray 16. In constructions in which the tibial bearing 14 is embodied as a fixed tibial bearing, the bearing 14 may or may not include the stem 36 and/or may include other devices or features to secure the tibial bearing 14 to the tibial tray 16 in a non-rotating configuration.

The upper bearing surface 32 of the tibial bearing 14 includes a medial bearing surface 42, a lateral bearing surface 44, and a spine 60 extending upwardly from the platform 18. The medial and lateral bearing surfaces 42, 44 are configured to receive or otherwise contact corresponding medial and lateral condyles 52, 54 of the femoral component 12 as discussed in more detail below. As such, each of the bearing surface 42, 44 has a concave contour. The spine 60 is positioned between the bearing surfaces 42, 44 and includes an anterior side 62 and a posterior side 64 having a cam surface 66. In the construction shown in the drawings, the cam surface 66 has a substantially concave curvature. However, spines 60 including cam surfaces 66 having other geometries may be used in other constructions. For example, a tibial bearing including a spine having a substantially "S"-shaped cross-sectional profile, such as the tibial bearing described in US-A-2009/326666, may be used.

The femoral component 12 is configured to be coupled to a surgically-prepared surface of the distal end of a patient's femur (not shown). The femoral component 12 may be secured to the patient's femur using bone adhesive or other attachment means. The femoral component 12 includes an outer, articulating surface 50 having a pair of medial and lateral condyles 52, 54. In use, the condyles 52, 54 replace the natural condyles of the patient's femur and are configured to articulate on the corresponding bearing surfaces 42, 44 of the platform 30 of the tibial bearing 14.

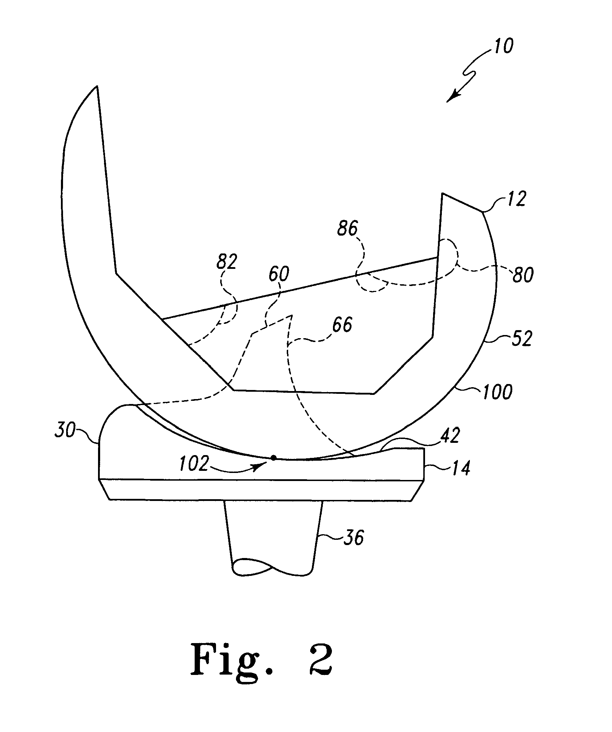

The condyles 52, 54 are spaced apart to define an intercondylar notch or recess 56 therebetween. A posterior cam 80 and an anterior cam 82 (see FIG. 2) are positioned in the intercondylar notch 56. The posterior cam 80 is located toward the posterior side of the femoral component 12 and includes a cam surface 86 is configured to engage or otherwise contact the cam surface 66 of the spine 60 of the tibial bearing 14 during flexion as shown in and described below with reference to FIGS. 2 to 4.

The orthopaedic knee prosthesis 10 shown in the drawings is configured to replace a patient's right knee and, as such, the bearing surface 42 and the condyle 52 are referred to as being medially located, and the bearing surface 44 and the condyle 54 are referred to as being laterally located. However, the orthopaedic knee prosthesis 10 may be configured to replace a patient's left knee. In such constructions, the bearing surface 42 and the condyle 52 may be laterally located and the bearing surface 44 and the condyle 54 may be medially located. Regardless, the features and concepts described herein may be incorporated in an orthopaedic knee prosthesis configured to replace either knee joint of a patient.

Referring now to FIGS. 2 to 4, the femoral component 12 is configured to articulate on the tibial bearing 14 during use. Each condyle 52, 54 of the femoral component 12 includes a condyle surface 100, which is convexly curved in the sagittal plane and configured to contact the respective bearing surface 42, 44. Additionally, during a predetermined range of flexion, the posterior cam 80 of the femoral component 12 contacts the spine 60 of the tibial bearing 14. For example, as shown in FIG. 2, when the orthopaedic knee prosthesis 10 is in extension or is otherwise not in flexion (e.g., a flexion of about 0 degrees), the condyle surface 100 of the condyle 52 contacts the bearing surface 42 (or bearing surface 44 in regard to condyle 54) at one or more contact points 102 on the condyle surface 100. Additionally, at this particular degree of flexion, the posterior cam 80 is not in contact with the spine 60. However, at later (i.e., larger) degrees of flexion, the posterior cam 80 is configured to contact the spine 60 to provide an amount of control over the kinematics of the orthopaedic prosthesis.

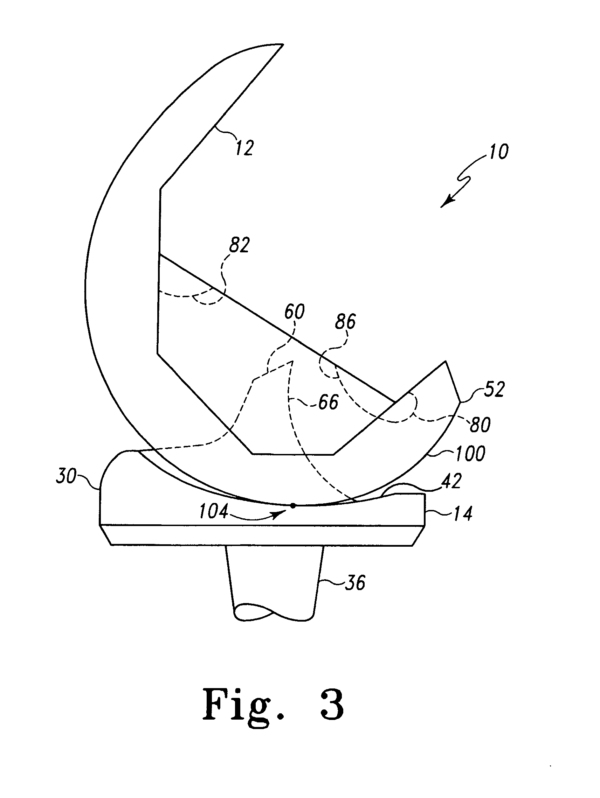

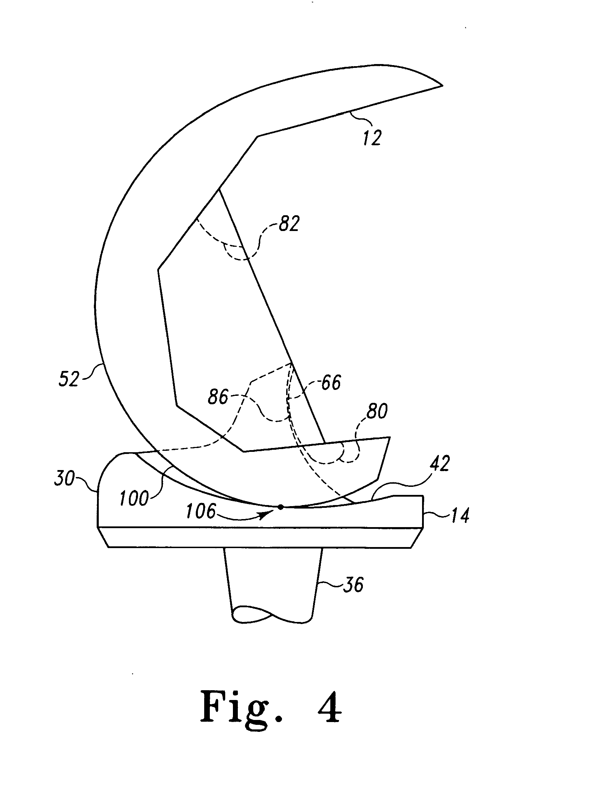

As the orthopaedic knee prosthesis 10 is articulated through the middle degrees of flexion, the femoral component 12 contacts the tibial bearing 14 at one or more contact points on the condyle surface 100. For example, as shown in FIG. 3, when the orthopaedic knee prosthesis 10 is articulated to a middle degree of flexion (e.g., at about 45 degrees), the condyle surface 100 contacts the bearing surface 42 at one or more contact points 104 on the condyle surface 100. As discussed in more detail below, depending on the particular construction, the posterior cam 80 may or may not be in contact with the spine 60 at this particular degree of flexion. Regardless, as the orthopaedic knee prosthesis 10 is articulated to a late degree of flexion (e.g., at about 70 degrees of flexion), the condyle surface 100 contacts the bearing surface 42 at one or more contact points 106 on the condyle surface 100 as shown in FIG. 4. Additionally, the posterior cam 80 is now in contact with the spine 60. It should be appreciated, of course, that the femoral component 12 may contact the tibial bearing 14 at a plurality of contact points on the condyle surface 100 at any one particular degree of flexion. However, for clarity of description, only the contact points 102, 104, 106 have been shown in FIGS.

2 to 4, respectively.

The particular degree of flexion at which the posterior cam 80 initially contacts the spine 60 is based on the particular geometry of the condyle surface 100 of the femoral component 12. For example, in the construction shown in FIGS. 2 to 4, the orthopaedic knee prosthesis 10 is configured such that the posterior cam 80 initially contacts the spine 60 at about 70 degrees of flexion. However, the posterior cam 80 may initially contact the spine 60 at other degrees of flexion as discussed below.

The orthopaedic knee prosthesis 10 is configured such that the amount of paradoxical anterior translation of the femoral component 12 relative to the tibial bearing 14 may be reduced or otherwise delayed to a later (i.e., larger) degree of flexion. In particular, as discussed below, the condyle surface 100 of one or both of the condyles 52, 54 has particular geometry or curvature configured to reduce and/or to delay anterior translations and, optionally, to promote "roll-back" or posterior translation, of the femoral component 12. By delaying the onset of paradoxical anterior translation of the femoral component 12 to a larger degree of flexion, the overall occurrence of paradoxical anterior translation may be reduced during those activities of a patient in which deep flexion is not typically obtained.

In a typical orthopaedic knee prosthesis, paradoxical anterior translation may occur whenever the knee prosthesis is positioned at a degree of flexion greater than zero degrees. The likelihood of anterior translation generally increases as the orthopaedic knee prosthesis is articulated to larger degrees of flexion, particularly in the mid-flexion range. In such orientations, paradoxical anterior translation of the femoral component on the tibial bearing can occur whenever the tangential (traction) force between the femoral component and the tibial bearing fails to satisfy the following equation:

$$T < \mu N \qquad\qquad (1)$$

in which "T" is the tangential (traction) force, "$\mu$" is the coefficient of friction of the femoral component and the tibial bearing, and "N" is the normal force between the femoral component and the tibial bearing. As a generalization, the tangential (traction) force between the femoral component and the tibial bearing can be defined as

$$T = M / R \qquad\qquad (2)$$

in which "T" is the tangential (traction) force between the femoral component and the tibial bearing, "M" is the knee moment, and "R" is the radius of curvature in the sagittal plane of the condyle surface in contact with the tibial bearing at the particular degree of flexion. It should be appreciated that equation (2) is a simplification of the governing real-world equations, which does not consider such other factors as inertia and acceleration. Regardless, the equation (2) provides insight that paradoxical anterior translation of an orthopaedic knee prosthesis may be reduced or delayed by controlling the radius of curvature of the condyle surface of the femoral component. That is, by controlling the radius of curvature of the condyle surface (e.g., increasing or maintaining the radius of curvature), the right-hand side of equation (2) may be reduced, thereby decreasing the value of the tangential (traction) force and satisfying the equation (1). As discussed above, by ensuring that the tangential (traction) force satisfies equation (1), paradoxical anterior translation of the femoral component on the tibial bearing may be reduced or otherwise delayed to a greater degree of flexion.

Based on the above analysis, to reduce or delay the onset of paradoxical anterior translation, the geometry of the condyle surface 100 of one or both of the condyles 52, 54 of the femoral component 12 is controlled. For example, the radius of curvature of the condyle surface 100 is controlled such that the radius of curvature is held constant over a range of degrees of flexion and/or is increased in the early to mid flexion ranges. Comparatively, typical femoral components have decreasing radii of curvatures beginning at the distal radius of curvature (i.e., at about 0 degrees of flexion). However, it has been determined that by maintaining a relatively constant radius of curvature (i.e., not decreasing the radius of curvature) over a predetermined range of degrees of early to mid-flexion and/or increasing the radius of curvature over the predetermined range of degrees of flexion may reduce or delay paradoxical anterior translation of the femoral component 12.

Additionally, in some constructions, the condyle surface 100 is configured or designed such that the transition between discrete radii of curvature of the condyle surface 100 is gradual. That is, by gradually transitioning between the discrete radii of curvature, rather than abrupt transitions, paradoxical anterior translation of the femoral component 12 may be reduced or delayed. Further, in some constructions, the rate of change in the radius of curvature of the condyle surface in the early to mid flexion ranges (e.g., from about 0 degrees to about 90 degrees) is controlled such that the rate of change is less than a predetermined threshold. That is, it has been determined that if the rate of change of the radius of curvature of the condyle surface 100 is greater than the predetermined threshold, paradoxical anterior translation may occur.

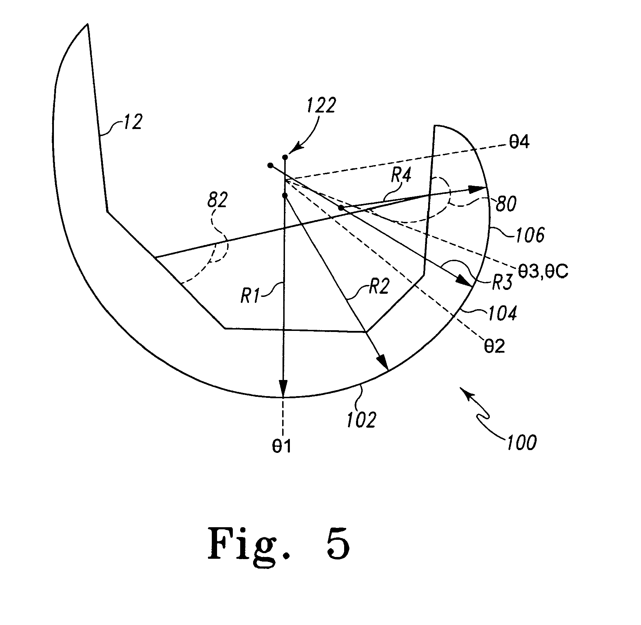

Accordingly, in the constructions shown in FIGS. 5 to 8, the condyle surface 100 of the femoral component 12 has an increased radius of curvature in early to middle degrees of flexion. By increasing the radius of curvature, paradoxical anterior translation may be reduced or delayed to a later degree of flexion as discussed in more detail below. In particular, paradoxical anterior translation may be delayed to a degree of flexion at or beyond which the posterior cam 80 of the femoral component 12 initially contacts the spine 60 of the tibial bearing 14. Once the posterior cam 80 is in contact with the spine 60, paradoxical anterior translation is controlled by the engagement of the posterior cam 80 to the spine 60. That is, the posterior cam 80 may be restricted from moving anteriorly by the spine 60.

The amount of increase between the radius of curvature R2 and the radius of curvature R3, as well as, the degree of flexion on the condyle surface 100 at which such increase occurs has been determined to affect the occurrence of paradoxical anterior translation. As discussed in more detail in US-A-2009/0326667, multiple simulations of various femoral component designs were performed using the LifeMOD/Knee Sim, version 1007.1.0 Beta 16 software program, which is commercially available from LifeModeler, Inc. of San Clemente, California, to analyse the effect of increasing the radius of curvature of the condyle surface of the femoral components in early and mid flexion. Based on such analysis, it has been determined that paradoxical anterior translation of the femoral component relative to the tibial bearing may be reduced or otherwise delayed by increasing the radius of curvature of the condyle surface by an amount in the range of about 0.5 mm to about 5 mm or more at a degree of flexion in the range of about 30 degrees of flexion to about 90 degrees of flexion.

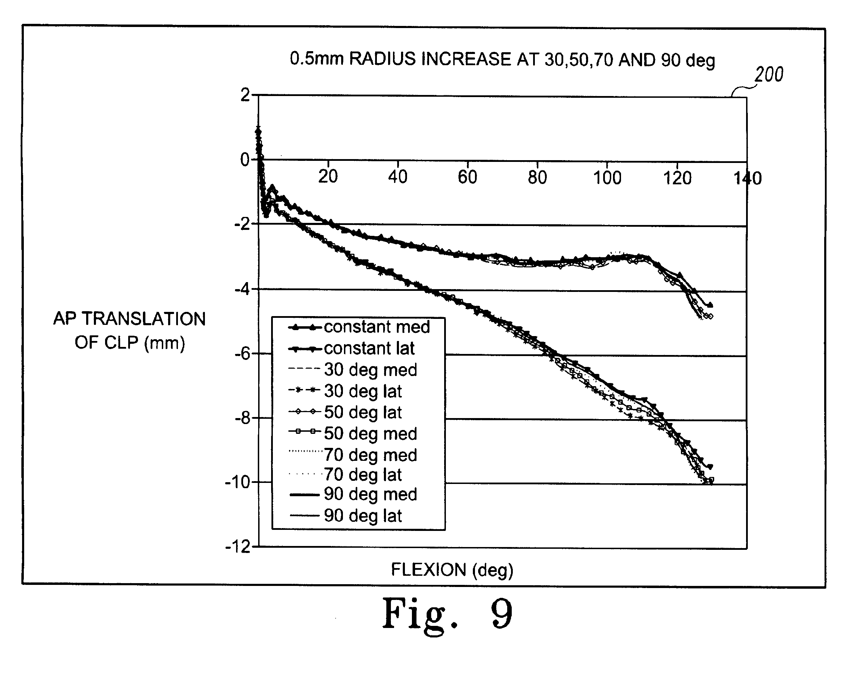

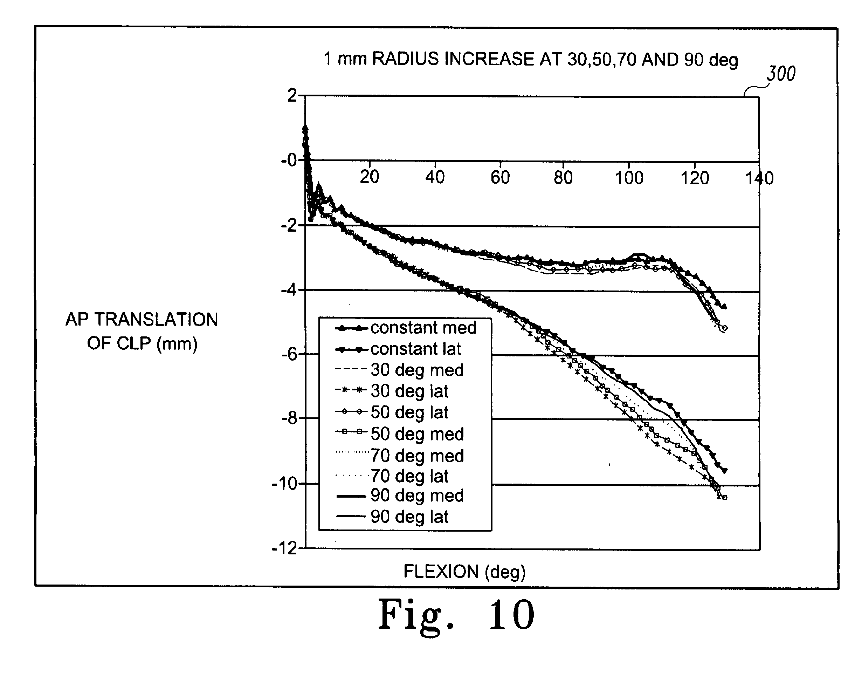

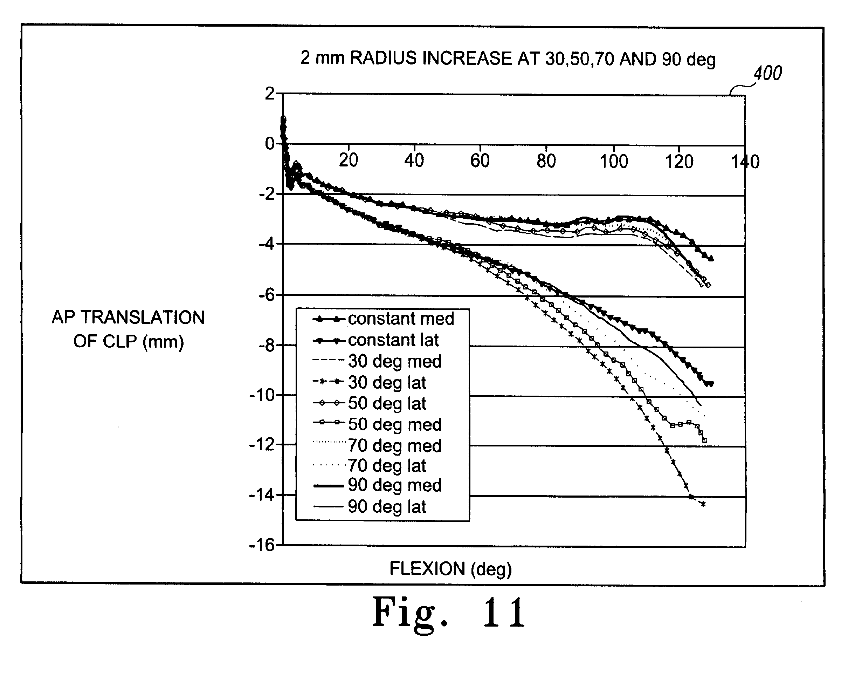

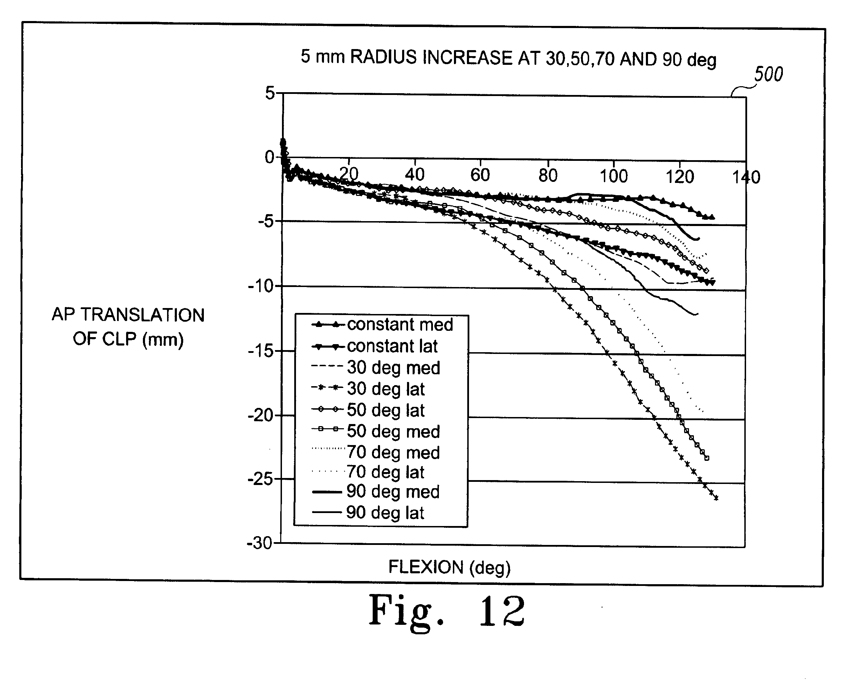

For example, the graph 200 in FIG. 9 presents the results of a deep bending knee simulation using a femoral component in which the radius of curvature of the condyle surface is increased by 0.5 mm (i.e., from 25.0 mm to 25.5 mm) at 30 degrees of flexion, at 50 degrees of flexion, at 70 degrees of flexion, and at 90 degrees of flexion. Similarly, the graph 300 in FIG. 10 presents the results of a deep bending knee simulation using a femoral component in which the radius of curvature of the condyle surface is increased by 1.0 mm (i.e., from 25.0 mm to 26.0 mm) at 30 degrees of flexion, at 50 degrees of flexion, at 70 degrees of flexion, and at 90 degrees of flexion. The graph 400 in FIG. 11 presents the results of a deep bending knee simulation using a femoral component in which the radius of curvature of the condyle surface is increased by 2.0 mm (i.e., from 25.0 mm to 27.0 mm) at 30 degrees of flexion, at 50 degrees of flexion, at 70 degrees of flexion, and at 90 degrees of flexion. Additionally, the graph 500 in FIG. 12 presents the results of a deep bending knee simulation using a femoral component in which the radius of curvature of the condyle surface is increased by 5.0 mm (i.e., from 25.0 mm to 26.0 mm) at 30 degrees of flexion, at 50 degrees of flexion, at 70 degrees of flexion, and at 90 degrees of flexion.

In the graphs 200, 300, 400, 500, the condylar lowest or most distal points (CLP) of the medial condyle ("med") and the lateral condyle ("lat") of the femoral component are graphed as a representation of the relative positioning of the femoral component to the tibial bearing. As such, a downwardly sloped line represents roll-back of the femoral component on the tibial bearing and an upwardly sloped line represents anterior translation of the femoral component on the tibial bearing.

As shown in the graphs 200, 300, 400, 500, anterior sliding of the femoral component was delayed until after about 100 degrees of flexion in each of the constructions; and the amount of anterior translation was limited to less than about 1 millimetre. In particular, "roll-back" of the femoral component on the tibial bearing was promoted by larger increases in the radius of curvature of the condyle surface at earlier degrees of flexion. Of course, amount of increase in the radius of curvature and the degree of flexion at which such increase is introduced is limited by other factors such as the anatomical joint space of the patient's knee, the size of the tibial bearing, and the like. Regardless, based on the simulations reported in the graphs 200, 300, 400, 500, paradoxical anterior translation of the femoral component on the tibial bearing can be reduced or otherwise delayed by increasing the radius of curvature of the condyle surface of the femoral component during early to mid flexion.

Accordingly, referring back to FIGS. 5 to 8, the condyle surface 100 in the sagittal plane is formed in part from a number of curved surface sections 102, 104, 106, 108 the sagittal ends of each of which are tangent to the sagittal ends of any adjacent curved surface section of the condyles surface 100. Each curved surface section 102, 104, 106, 108 is defined by a radius of curvature. In particular, the curved surface section 102 is defined by a radius of curvature R2, the curved surface section 104 is defined by a radius of curvature R3, the curved surface section 106 is defined by a radius of curvature R4.

The condyle surface 100 of the femoral component 12 is configured such that the radius of curvature R3 of the curved surface section 104 is greater than the radius of curvature R2 of the curved surface section 102. In one construction, the radius of curvature R3 is greater than the radius of curvature R2 by 0.5 mm or more. In another construction, the radius of curvature R3 is greater than the radius of curvature R2 by 2 mm or more. In another construction, the radius of curvature R3 is greater than the radius of curvature R2 by 2 mm or more. In a particular construction, the radius of curvature R3 is greater than the radius of curvature R2 by at least 5 mm or more. It should be appreciated, however, that the particular increase of radius of curvature between R2 and R3 may be based on or scaled to the particular size of the femoral component 12.

Each of the curved surface sections 102, 104, 106, 108 contacts the bearing surface 42 (or 44) of the tibial bearing 14 through different ranges of degrees of flexion. For example, the curved surface section 102 extends from an earlier degree of flexion θ1 to a later degree of flexion θ2. The curved surface section 104 extends from the degree of flexion θ2 to a later degree of flexion θ3. The curved surface section 106 extends from the degree of flexion θ3 to a later degree of flexion θ4.

For example, as shown in FIG. 5, the curved surface section 102 extends from a degree of flexion θ1 of about 0 degrees of flexion to a degree of flexion θ2 of about 50 degrees of flexion. The curved surface section 104 extends from the degree of flexion θ2 of about 50 degrees of flexion to a degree of flexion θ3 of about 70 degrees of flexion. The curved surface section 106 extends from the degree of flexion θ3 of about 70 degrees of flexion to a degree of flexion θ4 of about 120 degrees of flexion. In the construction shown in FIG. 5, the posterior cam 80 of the femoral component 12 is configured to engage or contact the spine 60 of the tibial bearing 14 at a degree of flexion θC of about 70 degrees of flexion. However, the posterior cam 80 may be configured to engage the spine 60 at a degree of flexion earlier or later than 70 degrees. By ensuring the posterior cam 80 engages or contacts the spine 60 prior to or soon after the reduction in radius of curvature from R3 to R4, the control of the kinematics of the orthopaedic prosthesis can be transitioned from the geometry of the condyle surface 100 to the interaction of the posterior cam 80 and spine 60, which may further reduce the amount of anterior translation of the femoral component 12. For example, in one particular construction, the posterior cam 80 may be configured to engage or contact the spine 60 at a degree of flexion θC that is no greater than 10 degrees more than the degree of flexion θ3 at which the radius curvature of the condyle surface 100 decreases from the radius of curvature R3 to the radius of curvature R4.

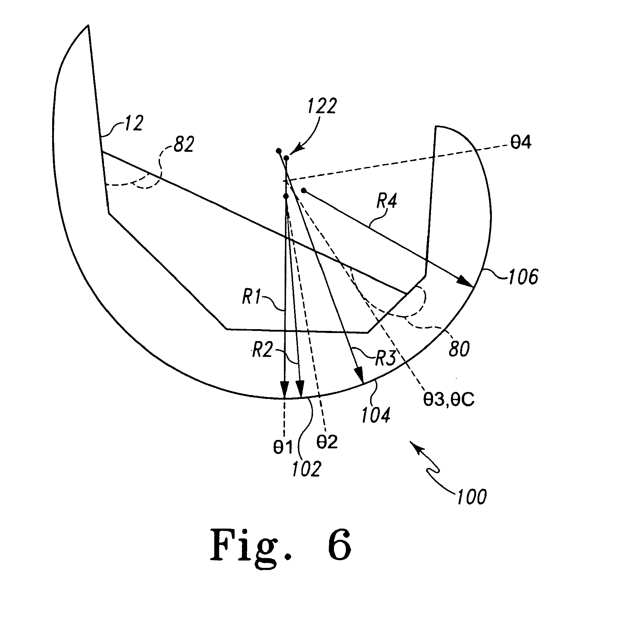

In another construction, as shown in FIG. 6, the curved surface section 102 extends from a degree of flexion θ1 of about 0 degrees of flexion to a degree of flexion θ2 of about 10 degrees of flexion. The curved surface section 104 extends from the degree of flexion θ2 of about 10 degrees of flexion to a degree of flexion θ3 of about 30 degrees of flexion. The curved surface section 106 extends from the degree of flexion θ3 of about 30 degrees of flexion to a degree of flexion θ4 of about 120 degrees of flexion. In the construction shown in FIG. 6, the posterior cam 80 of the femoral component 12 is configured to engage or contact the spine 60 of the tibial bearing 14 at a degree of flexion θC of about 30 degrees of flexion. Again, however, the posterior cam 80 may be configured to engage the spine 60 at a degree of flexion earlier than 30 degrees (i.e., earlier than the reduction in radius of curvature from R3 to R4) or soon thereafter (e.g., within 0 to 10 degrees).

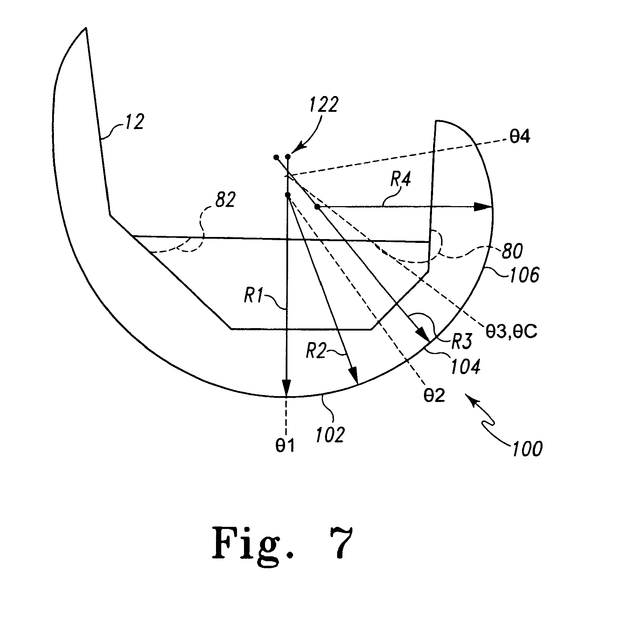

In another construction, as shown in FIG. 7, the curved surface section 102 extends from a degree of flexion θ1 of about 0 degrees of flexion to a degree of flexion θ2 of about 30 degrees of flexion. The curved surface section 104 extends from the degree of flexion θ2 of about 30 degrees of flexion to a degree of flexion θ3 of about 50 degrees of flexion. The curved surface section 106 extends from the degree of flexion θ3 of about 50 degrees of flexion to a degree of flexion θ4 of about 120 degrees of flexion. In the construction shown in FIG. 7, the posterior cam 80 of the femoral component 12 is configured to engage or contact the spine 60 of the tibial bearing 14 at a degree of flexion θC of about 50 degrees of flexion. Again, however, the posterior cam 80 may be configured to engage the spine 60 at a degree of flexion earlier than 50 degrees (i.e., earlier than the reduction in radius of curvature from R3 to R4) or soon thereafter (e.g., within 0 to 10 degrees).

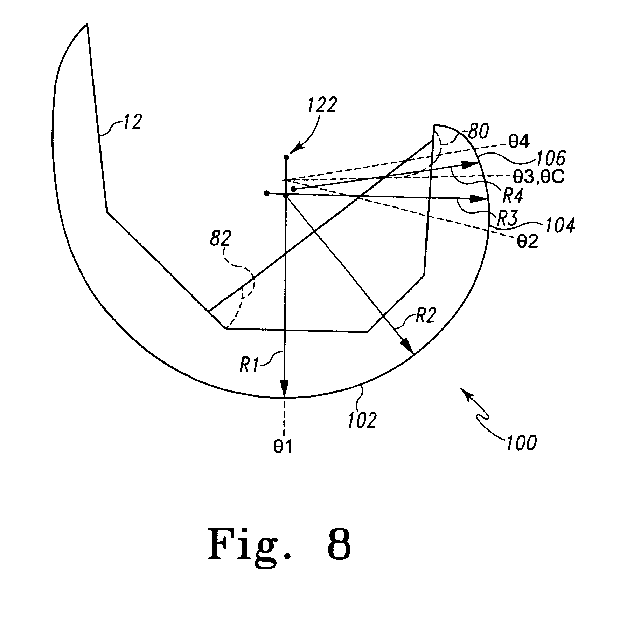

In another construction, as shown in FIG. 8, the curved surface section 102 extends from a degree of flexion θ1 of about 0 degrees of flexion to a degree of flexion θ2 of about 70 degrees of flexion. The curved surface section 104 extends from the degree of flexion θ2 of about 70 degrees of flexion to a degree of flexion θ3 of about 90 degrees of flexion. The curved surface section 106 extends from the degree of flexion θ3 of about 90 degrees of flexion to a degree of flexion θ4 of about 120 degrees of flexion. In the construction shown in FIG. 8, the posterior cam 80 of the femoral component 12 is configured to engage or contact the spine 60 of the tibial bearing 14 at a degree of flexion θC of about 90 degrees of flexion. Again, however, the posterior cam 80 may be configured to engage the spine 60 at a degree of flexion earlier than 90 degrees (i.e., earlier than the reduction in radius of curvature from R3 to R4) or soon thereafter (e.g., within 0 to 10 degrees).

Each of the curved surface sections 102, 104, 106 may extend from degrees of flexion different from those shown and discussed above with reference to FIGS. 5 to 8. For example, in each of the constructions shown in FIGS. 5 to 8, although the curved surface section 102 is shown as beginning at about 0 degrees of flexion, the curved surface section 102 may being at a degree of flexion prior to 0 degrees of flexion (i.e., a degree of hyperextension).

Additionally, the degree of flexion θC at which the posterior cam 80 contacts the spine 60 may be less than, substantially equal to, or slightly greater than the degree of flexion θ3 at which the radius of curvature R3 decreases to the radius of curvature R4. In some constructions, the degree of flexion θC is within a predetermined threshold of the degree of flexion θ3. For example, the degree of flexion θC is within about 10 degrees of the degree of flexion θ3. For example, the radius of curvature R3 may decrease to the radius of curvature R4 at a degree of flexion θ3 of about 70 degrees and the posterior cam 80 may be configured to initially contact the spine 60 at a degree of flexion θC of in the range of about 60 to about 80 degrees of flexion.

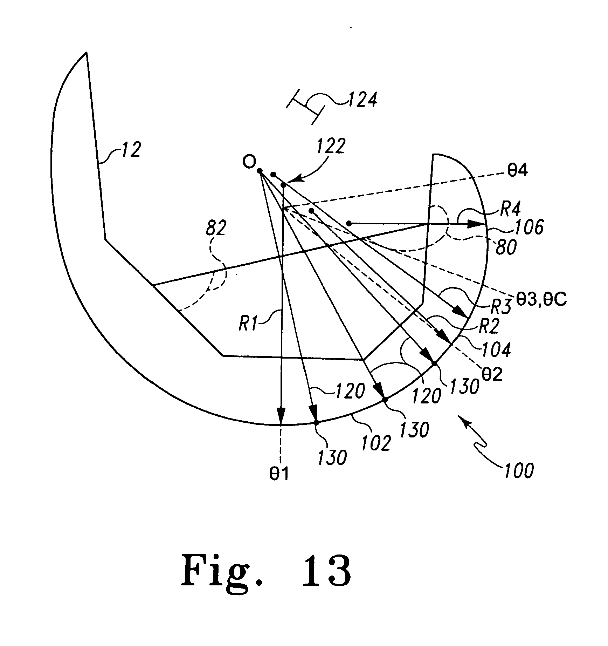

Referring now to FIGS. 13 to 15, the condyle surface 100 may include a gradual transition between discreet radii of curvature in the early to mid flexion ranges such that the change in the radius of curvature of the condyle surface over a range of degrees of flexion is reduced. For example, as shown in FIG. 13, the curved surface section 102 in some constructions is designed to provide a gradual transition from the first radius of curvature R1 to the second radius of curvature R2. To do so, the curved surface section 102 is defined by a plurality of rays 120 rather than a constant radius of curvature as illustrated in and described above with reference to FIGS. 5 to 8. Each of the plurality of rays 120 originate from a common origin O. Additionally, each of the plurality of rays 120 defines a respective contact point 130 on the curved surface section 102. Although only three rays 120 are shown in FIG. 13 for clarity of the drawing, an infinite number of rays 120 may be used to define the curved surface section 102.

The location of each contact points 130, which collectively define the curved surface section 102, can be determined based on the length of each ray 120 at each degree of flexion. In particular and unexpectedly, it has been determined that paradoxical anterior translation of the femoral component 12 on the tibial bearing 14 may be reduced or delayed by defining the curved surface section 102 according to the following polynomial equation:

$$r_\theta = (a + (b * \theta) + (c * \theta^2) + (d * \theta^3)), \qquad (3)$$

in which "$r_\theta$" is the length of a ray 120 (in metric units) defining a contact point 130 on the curved surface section 104 at "$\theta$" degrees of flexion, "a" is a scalar value between 20 and 50, and "b" is a coefficient value selected such that:

$$-0.30 < b < 0.00, \qquad (4)$$
$$0.00 < b < 0.30, \text{ or}$$
$$b = 0$$

If the selected coefficient "b" is in the range of -0.30 < b < 0.00, then coefficients "c" and "d" are selected such that:

$$0.00 < c < 0.012, \text{ and} \qquad (5)$$
$$-0.00015 < d < 0.00.$$

Alternatively, if the selected coefficient "b" is in the range of 0.00 < b < 0.30, then coefficients "c" and "d" are selected such that:

$$-0.010 < c < 0.00, \text{ and} \qquad (6)$$
$$-0.00015 < d < 0.00.$$

Further, if the selected coefficient "b" is equal to 0, then coefficients "c" and "d" are selected such that:

$$-0.0020 < c < 0.00, \text{ or} \qquad (7)$$
$$0.00 < c < 0.0025, \text{ and}$$
$$-0.00015 < d < 0.00.$$

Ranges of values for the scalar "a" and coefficients "b", "c", and "d" have been determined from an infinite number of possible solutions for the polynomial equation (3). That is, the particular set of ranges provided above have been determined to generate a family of curves (i.e., the curved surface section 102) that provide a gradual transitioning of the condyle surface 100 from the radius of curvature R1 to the radius of curvature R2 such that anterior translation of the femoral component 12 relative to the tibial bearing 14 is reduced or delayed. Additionally, the range of values for each coefficient "a", 'b", "c", and "d" are provided above in regard to constructions designed using the metric system of units. However, such range of coefficient values may be converted for use in constructions using other systems of units such as the English system of units.

The overall shape of the curved surface section 102 is also affected by the placement of the common origin O of the plurality of rays 120. By limiting the distance 124 between the common origin O of the plurality of rays 120 and the origin 122 of the distal radius of curvature R1, paradoxical anterior sliding of the femoral component 12 on the tibial bearing 14 may be reduced or delayed. Additionally, stability of the orthopaedic knee prosthesis 10 may be improved by ensuring the common origin O of the plurality of rays 120 is within the predetermined distance 124 from the origin 122 of the distal radius of curvature R1. As such, in one construction, the location of the common origin O of the plurality of rays 120 is selected such that the distance 124 between the common origin O and the origin 122 of the radius of curvature R1 is less than about 10 mm to reduce or delay anterior translation of the femoral component and/or provide improved stability to the orthopaedic knee prosthesis 10.

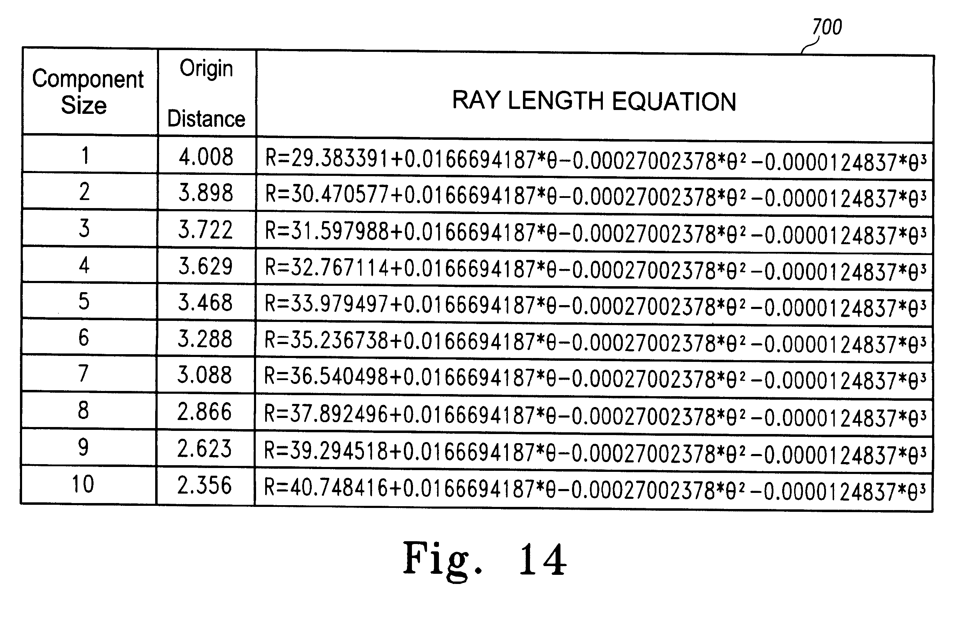

The distance 124 between the common origin O and the origin 122 of the radius of curvature R1 and the particular coefficient values may be dependent upon the particular size of the femoral component 12. For example, as shown in FIG. 14, a table 700 contains a set of coefficient values for the above-defined polynomial equation (3) and values for the distance 124 defined between the common origin O and the origin 122 of the distal radius of curvature R1. As shown in table 700, the distance 124 between the common origin O and the origin 122 of the radius of curvature R1 and the value for the scalar "a" change across the femoral component sizes. However, in this particular construction, the values for the coefficients "b", "c", and "d" are constant across the femoral component sizes. In other constructions, the coefficient values "b", "c", and "d" may change across the femoral component sizes.

As discussed above, in some construction, the condyle surface 100 is further designed or configured such that the change in the radius of curvature of the condyle surface 100 in the early and mid flexion ranges is not too great or too abrupt (e.g., the ratio of the degree of change in radius of curvature to the change in degrees of flexion is too great). That is, if the ratio of the radius of curvature R1 to the radius of curvature R2, R3, or R4 is too great, paradoxical anterior translation of the femoral component 12 may occur. As such, by designing the condyle surface 100 of the femoral component 12 such that the ratios of the distal radius of curvature R1 to (i) the radius of curvature R2 of the curved surface section 102, (ii) the radius of curvature R3 of the curved surface section 104, and (iii) the radius of curvature R4 of the late flexion curved surface section 106 are less than a predetermined threshold value, paradoxical anterior sliding may unexpectedly be reduced or otherwise delayed.

Accordingly, in one particular construction, the condyle surface 100 of the femoral component 12 is designed such that the ratio of the radius of curvature of R1 to the radius of curvature of R2 is between about 1.10 to about 1.30, the ratio of the radius of curvature of R1 to the radius of curvature R3 is between about 1.001 to about 1.100, and the ratio of the radius of curvature of R1 to the radius of curvature R4 is about 1.25 to about 2.50. Further, in some constructions, the ratio of the radius of curvature of R2 to the radius of curvature of R3 is between about 0.74 and about 0.85.

The particular amount of increase in the radius of curvature R2 to R3 of the condyle surface 100 of the femoral component 12 and/or the positioning of such increase on the condyle surface 100 may also be based on, scaled, or otherwise affected by the size of the femoral component 12. That is, an increase of the radius of curvature R2 to R3 of the condyle surface 100 of 0.5 mm is a relatively larger increase in small-sized femoral components compared to larger-sized femoral components. As such, the magnitude of the increase in the radius of curvature R2 to R3 of the condyle surface 100 of the femoral component 12 may change across femoral component sizes. In one construction, however, the ratios of the radius of curvatures R1 to the radius of curvatures R2, R3, and R4 are maintained at a substantially constant value across the family of femoral component sizes.

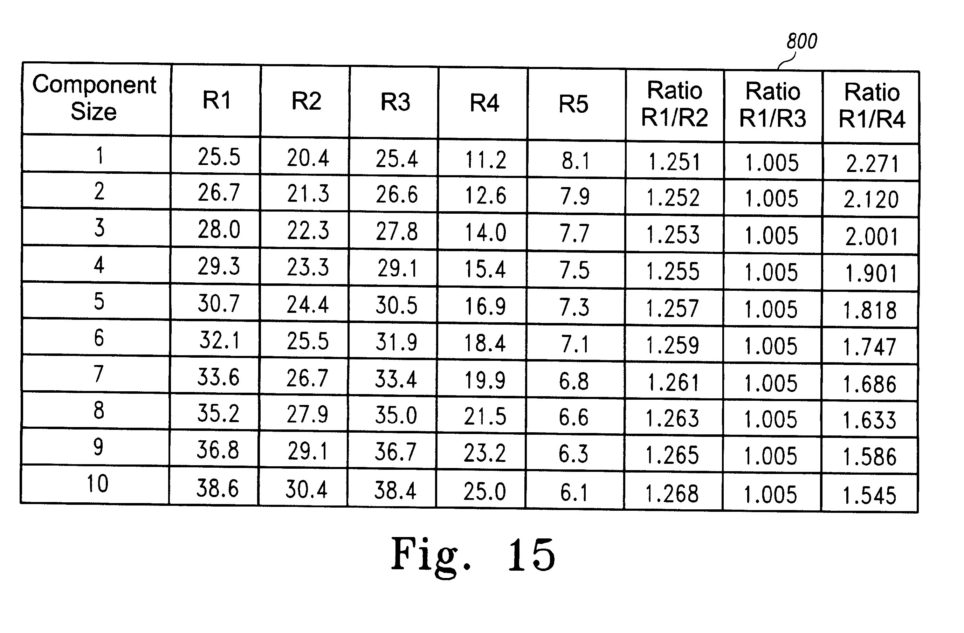

For example, as shown in FIG. 15, a table 800 defines the length of each radius of curvature R1, R2, R3, R4 for a family of femoral component sizes 1 through 10. As set out in the table 850, the length of each radius of curvature R1, R2, R3, R4 for each size 1 to 10 of the femoral component 12 is selected such that the ratios of R1/R2 and R1/R3 are substantially constant across the femoral component sizes. In this construction, as previously discussed, the ratio of the radius of curvature R1 to the radius of curvature R2 is maintained at a value of about 1.25 to about 1.27 across the femoral component sizes 1 through 10 and the ratio of the radius of curvature R1 to the radius of curvature R3 is maintained at a value of about 1.005 across the femoral component sizes 1 to 10.

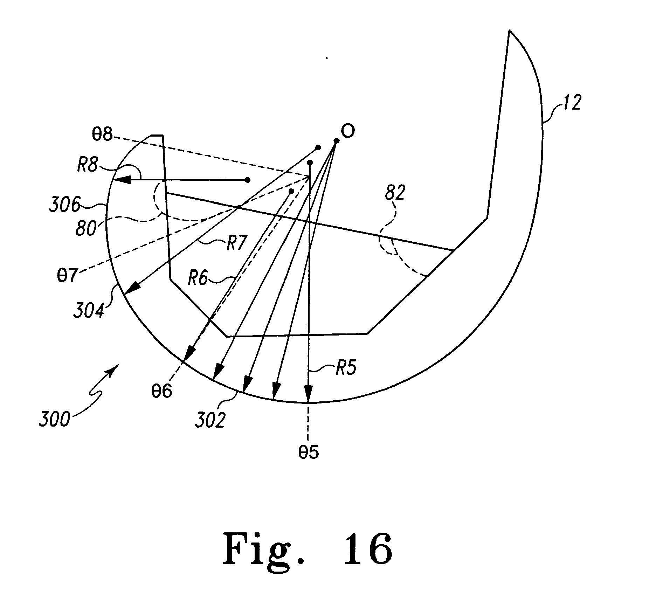

The overall shape and design of the condyle surface 100 of the femoral component 12 has been described above in relation to a single condyle 52, 54 of the femoral component 12. In some constructions, both condyles 52, 54 of the femoral component 12 may be symmetrical and have similar condyle surfaces 100. However, the condyles 52, 54 of the femoral component 12 may be asymmetrical. For example, as shown in FIG. 16, the femoral component 12 may include a second condyle 52, 54 having a condyle surface 300, which is defined in part by a plurality of curved surface sections 302, 304, 306. The curved surface section 302 extends from an earlier degree of flexion θ5 to a later degree of flexion θ6. The curved surface section 304 extends from the degree of flexion θ6 to a later degree of flexion θ7. The curved surface section 306 extends from the degree of flexion θ7 to a later degree of flexion θ8. The condyle surface 300 also includes a distal radius R5, which is gradually transitioned to a radius of curvature R6 via the curved surface section 302. Additionally, the curved section 304 is defined by a radius of curvature R7 and the curved section 306 is defined by a radius of curvature R8.

As such, when the condyles 52, 54 are symmetrical, the degree of flexion θ5 is substantially equal to the degree of flexion θ1, the degree of flexion θ6 is substantially equal to the degree of flexion θ2, the degree of flexion θ7 is substantially equal to the degree of flexion θ3, and the degree of flexion θ8 is substantially equal to the degree of flexion θ4. Additionally, the radius of curvature R5 is substantially equal to the radius of curvature R1, the radius of curvature R6 is substantially equal to the radius of curvature R2, the radius of curvature R7 is substantially equal to the radius of curvature R3, and the radius of curvature R8 is substantially equal to the radius of curvature R4. Further, the set of coefficient values "a", b", "c", and/or "d" of the equation (4) described above are substantially similar for both condyles.

However, in other constructions, the condyles 52, 54 are asymmetrical. As such, the degree of flexion θ5 may be different from the degree of flexion θ1. Additionally, the degree of flexion θ6 may be different from the degree of flexion θ2. That is, the increase in radius of curvature between R2 and R3 may occur at different degrees of flexion between the condyles 52, 54. Further, the degree of flexion θ8 may be different from the degree of flexion θ4. However, the degree of flexion θ7 may be substantially equal to the degree of flexion θ3 such that the posterior cam 80 is positioned properly within the intercondylar notch 56.

Additionally, when the condyles 52, 54 are asymmetrical, the radius of curvature R5 may be different from the radius of curvature R1, the radius of curvature R6 may be different from the radius of curvature R2, the radius of curvature R7 may be different from the radius of curvature R3, and/or the radius of curvature R8 may be different from the radius of curvature R4. Further, the set of coefficient values "a", b", "c", and/or "d" of the equation (3) described above may be different between the condyle surfaces 100 and 300.

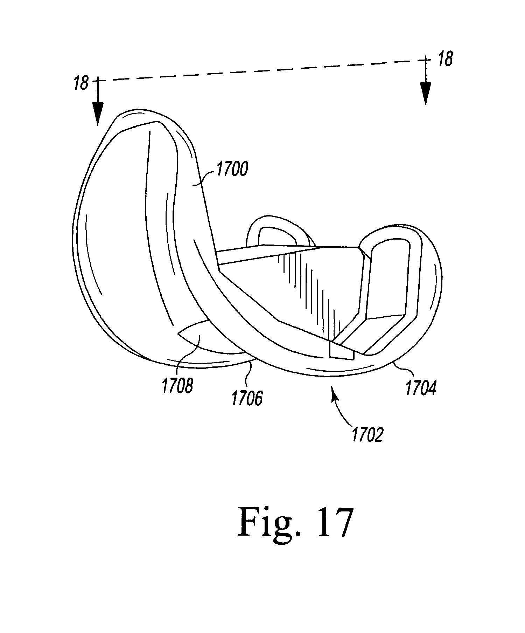

In another construction, the femoral component 12 of the orthopaedic knee prosthesis 10 may be embodied as a femoral component 1700 as shown in FIGS. 17 to 22, which is similar to the femoral component 12. The femoral component 1700 is configured to be coupled to a surgically-prepared surface of the distal end of a patient's femur (not shown). The femoral component 1700 may be secured to the patient's femur via use of bone adhesive or other attachment means. The femoral component 1700 includes an outer, articulating surface 1702 having a pair of medial and lateral condyles 1704, 1706. In use, the condyles 1704, 1706 replace the natural condyles of the patient's femur and are configured to articulate on the corresponding bearing surfaces 42, 44 of the platform 30 of the tibial bearing 14.

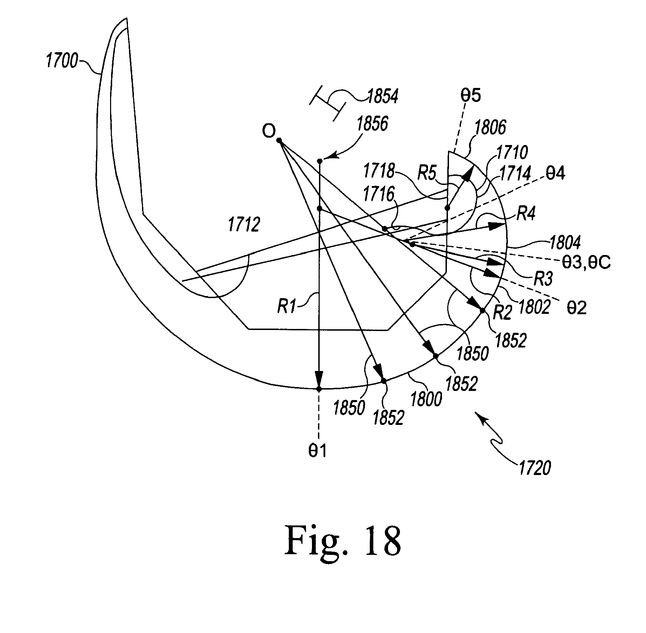

The condyles 1704, 1706 are spaced apart to define an intercondylar notch or recess 1708 therebetween. A posterior cam 1710 and an anterior cam 1712 (see FIG. 18) are positioned in the intercondylar notch 1708. The posterior cam 1710 is located toward the posterior side of the femoral component 1700 and includes a cam surface 1714 is configured to engage or otherwise contact the cam surface 66 of the spine 60 of the tibial bearing 14 during flexion. Illustratively, the cam surface 1714 as a substantial "S-shaped" sagittal cross-section and includes a concave cam surface 1716 and a convex cam surface 1718 similar to the posterior cam described in US-A-2009/0326666. However, the cam surface 1714 may have a simpler geometry such as the convex cam surface geometry of the femoral component 12 illustrated in FIG. 2.

As discussed above with reference to the femoral component 12, the femoral component 1700 is configured to articulate on the tibial bearing 14 during use. Each condyle 1704, 1706 of the femoral component 1700 includes a condyle surface 1720, which is convexly curved in the sagittal plane and configured to contact the respective bearing surface 42, 44. Additionally, during a predetermined range of flexion, the posterior cam 1710 of the femoral component 1700 contacts the spine 60 of the tibial bearing 14.

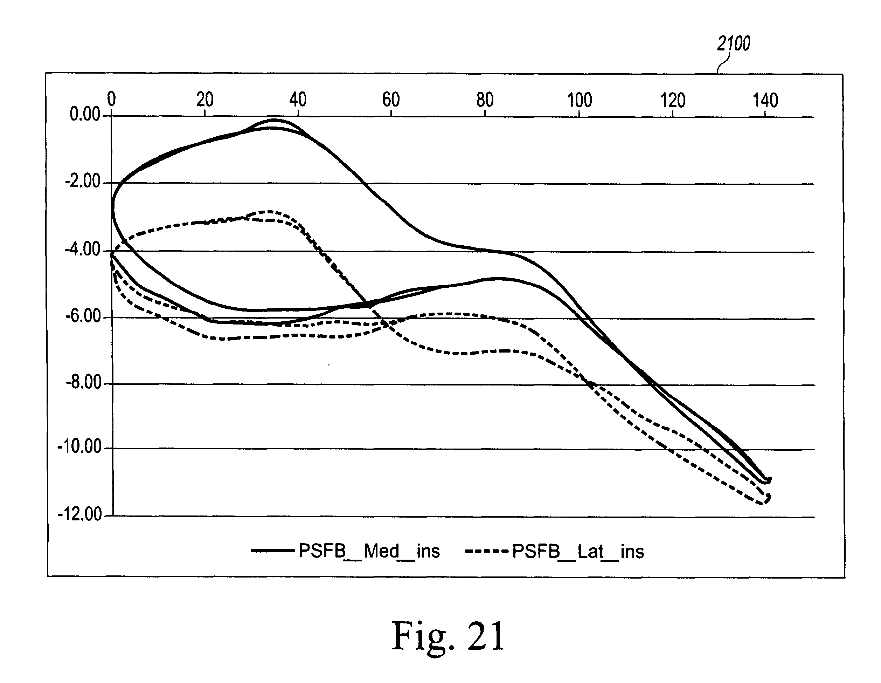

As discussed above, the orthopaedic knee prosthesis 10 is configured such that the amount of paradoxical anterior translation of the femoral component 1700 relative to the tibial bearing 14 may be reduced or otherwise delayed to a later (i.e., larger) degree of flexion. To do so, the condyle surface 1720 of one or both of the condyles 1704, 1706 has particular geometry or curvature configured to reduce and/or delay anterior translations and, in some constructions, promote "roll-back" or posterior translation, of the femoral component 1700. By delaying the onset of paradoxical anterior translation of the femoral component 1700 to a larger degree of flexion, the overall occurrence of paradoxical anterior translation may be reduced during those activities of a patient in which deep flexion is not typically obtained. In particular, paradoxical anterior translation may be delayed to a degree of flexion at or beyond which the posterior cam 1710 of the femoral component 1700 initially contacts the spine 60 of the tibial bearing 14. Once the posterior cam 1710 is in contact with the spine 60, paradoxical anterior translation is controlled by the engagement of the posterior cam 1710 to the spine 60. That is, the posterior cam 1710 may be restricted from moving anteriorly by the spine 60. For example, the graph 2100 in FIG. 21 presents the results of a deep bending knee simulation using a femoral component in which the initial degree of flexion at which the posterior cam 1710 of the femoral component 1700 contacts the spine 60 of the tibial bearing at 30 degrees of flexion, at 50 degrees of flexion, at 70 degrees of flexion, and at 90 degrees of flexion.

As shown in FIG. 18, the condyle surface 1720 in the sagittal plane is formed in part from a number of curved surface sections 1800, 1802, 1804, 1806 the sagittal ends of each of which are tangent to the sagittal ends of any adjacent curved surface section of the condyle surface 1720. Each of the curved surface sections 1800, 1802, 1804, 1806 contacts the bearing surface 42 (or 44) of the tibial bearing 14 through different ranges of degrees of flexion. For example, the curved surface section 1800 extends from an earlier degree of flexion θ1 to a later degree of flexion θ2. The curved surface section 1802 extends from the degree of flexion θ2 to a later degree of flexion θ3. The curved surface section 1804 extends from the degree of flexion θ3 to a later degree of flexion θ4. The curved surface section 1806 extends from the degree of flexion θ4 to a later degree of flexion θ5.

For example, as shown in FIG. 18, the curved surface section 1800 extends from a degree of flexion θ1 of about 0 degrees of flexion to a degree of flexion θ2 of about 70 degrees of flexion. However, the degree of flexion θ2 may range from slightly greater than θ1 to about 75 degrees. The curved surface section 1802 illustratively extends from the degree of flexion θ2 of about 73 degrees of flexion to a degree of flexion θ3 of about 73 degrees. However, the degree of flexion θ3 may range from about 73 degrees to about 90 degrees. The curved surface section 1804 illustratively extends from the degree of flexion θ3 of about 73 degrees of flexion to a degree of flexion θ4 of about 120 degrees of flexion. However, the degree of flexion θ4 may range from about 90 degrees to about 120 degrees. The curved surface section 1806 illustratively extends from the degree of flexion θ4 of about 120 degrees of flexion to a degree of flexion θ5 of about 165 degrees of flexion. However, the degree of flexion θ5 may range from about 140 degrees to about 165 degrees.

In the construction shown in FIG. 17, the posterior cam 1710 of the femoral component 1700 is configured to engage or contact the spine 60 of the tibial bearing 14 at a degree of flexion θC near or within the range of flexions of θ2 and θ3. For example, the posterior cam 1710 may initially engage the spine 60 at a degree of flexion θC of about 70 degrees to about 80 degrees. In one particular construction, the posterior cam 1710 is configured to initially engage the spine 60 t a degree of flexion θC of about 73 degrees. By ensuring the posterior cam 1710 engages or contacts the spine 60 prior to or soon after the posterior end of the curved surface section 1800, the control of the kinematics of the orthopaedic prosthesis can be transitioned from the geometry of the condyle surface 1720 to the interaction of the posterior cam 1710 and spine 60, which may further reduce the amount of anterior translation of the femoral component 1700.

Each of the curved surface sections 1802, 1804, 1806 is defined by a substantially constant radius of curvature, whereas the curved surface section 1800 is defined by a non-constant radius of curvature. That is, the curved surface section 1800 has a radius of curvature that begins with R1 at θ1 and gradually decreases to R2 at θ2 (i.e., R1 < R2). Conversely, the curved surface section 1802 is defined by a substantially constant radius of curvature R3, the curved surface section 1804 is defined by a substantially constant radius of curvature R4, and the curved surface section 1806 is defined by a substantially constant radius of curvature R5. In the construction shown in the drawings, the condyle surface 1720 is configured such that the radius of curvature R3 is less than or equal to the radius of curvature R2. Additionally, the ratio of the radius of curvature R4 to the radius of curvature R3 is configured to be in the range of about 0.7 to about 1.15 in some constructions. Further, the radius of curvature R5 is less than the radius of curvature R4. However, that the particular relationship between radii of curvature of the condyle surface 1720 may vary based on the particular size of the femoral component 12.

As discussed above, the initial curved surface section 1800 is designed to provide a gradual transition from the first radius of curvature R1 to the second radius of curvature R2. To do so, the curved surface section 1800 is defined by a plurality of rays 1850 rather than a constant radius of curvature. Each of the plurality of rays 1850 originate from a common origin O. Additionally, each of the plurality of rays 1850 defines a respective contact point 1852 on the curved surface section 1800. Although only three rays 1850 are illustrated in FIG. 18 for clarity of the drawing, it should be appreciated that an infinite number of rays 1850 may be used to define the curved surface section 1800.

The location of each contact points 1852, which collectively define the curved surface section 1800, can be determined based on the length of each ray 1850 at each degree of flexion. In particular and unexpectedly, it has been determined that paradoxical anterior translation of the femoral component 12 on the tibial bearing 14 may be reduced or delayed by defining the curved surface section 1800 according to the following polynomial equation:

$$r_\theta \;=\; (a \;+\; (b * \theta) \;+\; (c * \theta^2) \;+\; (d * \theta^3)), \qquad\qquad (8)$$

in which "$r_\theta$" is the length of a ray 1850 (in metric units) defining a contact point 1852 on the curved surface section 1800 at "θ" degrees of flexion, "a" is a scalar value between 35 and 45, and "b" is a coefficient value selected such that:

$$0.00 \;<\; b \;<\; 0.30, \text{ or} \qquad\qquad (9)$$
$$b = 0.015384615$$

If the selected coefficient "b" is in the range of 0.00 < b < 0.30, then coefficients "c" and "d" are selected such that:

$$-0.010 \;<\; c \;<\; 0.000, \text{ and} \qquad\qquad (10)$$
$$-0.00015 \;<\; d \;<\; 0.00.$$

Alternatively, if the selected coefficient "b" is equal to 0.015384615, then coefficients "c" and "d" are selected such that:

$$c = -0.00027024, \text{ and} \qquad (11)$$

$$d = -0.0000212$$

Ranges of values for the scalar "a" and coefficients "b", "c", and "d" have been determined from an infinite number of possible solutions for the polynomial equation (8). That is, the particular set of ranges provided above have been determined to generate a family of curves (i.e., the curved surface section 1800) that provide a gradual transitioning of the condyle surface 1720 from the radius of curvature R1 to the radius of curvature R2 such that anterior translation of the femoral component 1700 relative to the tibial bearing 14 is reduced or delayed. Additionally, the range of values for each coefficient "a", 'b", "c", and "d" are provided above in relation to constructions designed using the metric system of units. However, such range of coefficient values may be converted for use in constructions using other systems of units such as the English system of units.

The overall shape of the curved surface section 1800 is also affected by the placement of the common origin O of the plurality of rays 1850. By limiting the distance 1854 between the common origin O of the plurality of rays 1850 and the origin 1856 of the distal radius of curvature R1, paradoxical anterior sliding of the femoral component 1700 on the tibial bearing 14 may be reduced or delayed. Additionally, stability of the orthopaedic knee prosthesis 10 may be improved by ensuring the common origin O of the plurality of rays 1850 is within the predetermined distance 1854 from the origin 1856 of the distal radius of curvature R1. As such, in one construction, the location of the common origin O of the plurality of rays 1850 is selected such that the distance 1854 between the common origin O and the origin 1856 of the radius of curvature R1 is less than about 10 mm to reduce or delay anterior translation of the femoral component and/or provide improved stability to the orthopaedic knee prosthesis 10.

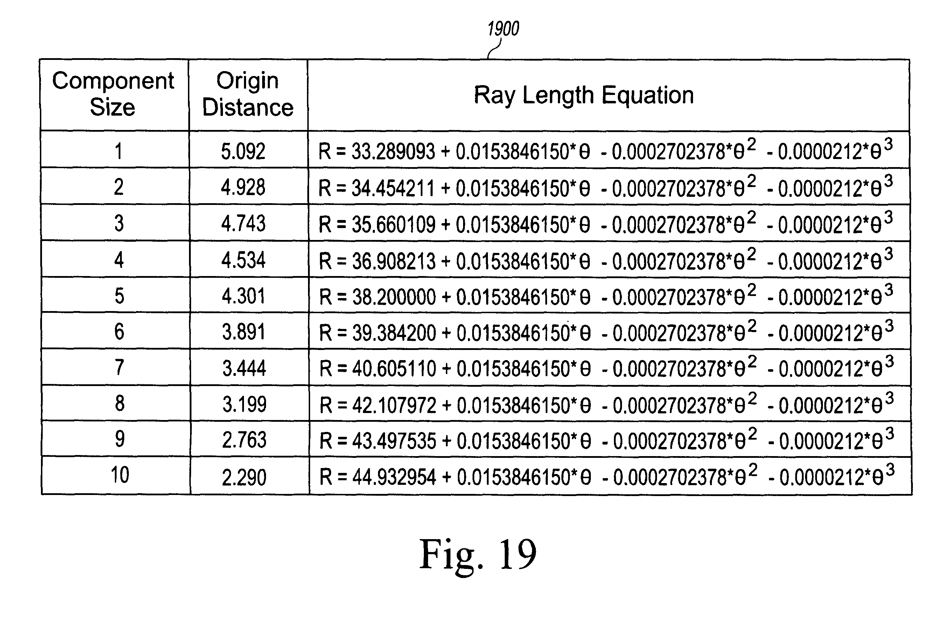

The distance 1854 between the common origin O and the origin 1856 of the radius of curvature R1 and the particular coefficient values may be dependent upon the particular size of the femoral component 1700. For example, as shown in FIG. 19, a table 1900 contains coefficient values for the above-defined polynomial equation (8) and values for the distance 1854 defined between the common origin O and the origin 1856 of the distal radius of curvature R1. As shown in table 1900, the distance 1854 between the common origin O and the origin 1856 of the radius of curvature R1 and the value for the scalar "a" change across the femoral component sizes. However, in this particular construction, the values for the coefficients "b", "c", and "d" are constant across the femoral component sizes. However, the coefficient values "b", "c", and "d" may change across the femoral component sizes.

In some constructions, the condyle surface 1720 is further designed or configured such that the change in the radius of curvature of the condyle surface 1720 in the early and mid flexion ranges is not too great or too abrupt (e.g., the ratio of the degree of change in radius of curvature to the change in degrees of flexion is too great). That is, if the ratio of the radius of curvatures of adjacent curved surface sections 1800, 1802, 1804, 1806 is too great, paradoxical anterior translation of the femoral component 1700 may occur. As such, by designing the condyle surface 1720 of the femoral component 1700 such that the ratio of the distal radius of curvature R1 to the radius of curvature R2 of the curved surface section 1800, (ii) the radius of curvature R2 to the radius of curvature R3 of the curved surface section 1802, (iii) the radius of curvature R3 to the radius of curvature R4 of the curved surface section 1804, and (iv) the radius of curvature R4 to the radius of curvature R5 of the curved surface section 1806 are less than a predetermined threshold value, paradoxical anterior sliding may unexpectedly be reduced or otherwise delayed.

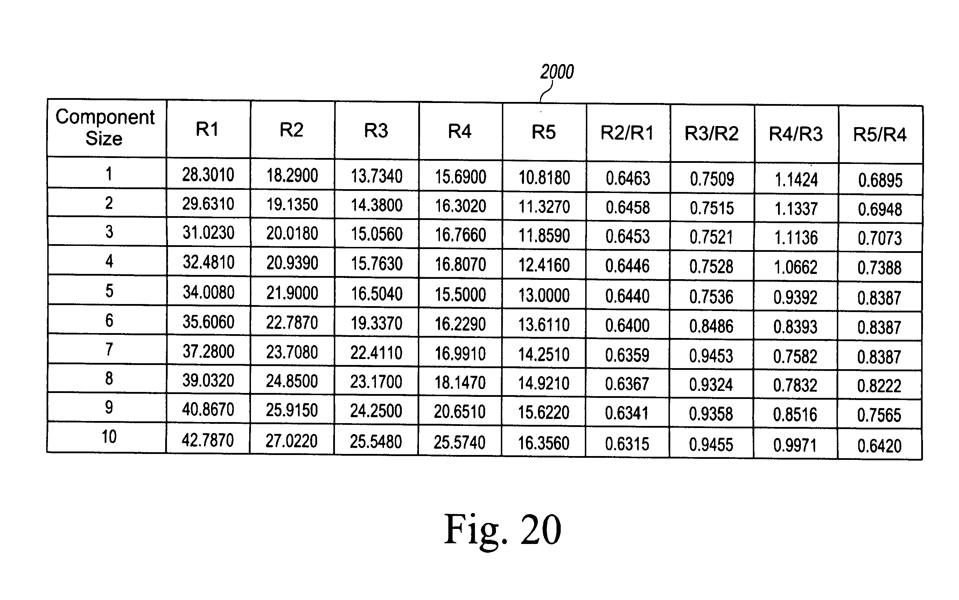

Accordingly, in one particular construction, the condyle surface 1720 of the femoral component 1700 is designed such that the ratio of the radius of curvature of R2 to the radius of curvature of R1 is between about 0.6 to about 0.7, the ratio of the radius of curvature R3 to the radius of curvature R2 is between about 0.7 and about 1.0, the ratio of the radius of curvature R4 to the radius of curvature R3 is between about 0.7 to about 1.15, and the radius of curvature R5 to the radius of curvature R4 is between about 0.6 to about 0.9. For example, as shown in FIG. 20, a table 2000 defines the length of each radius of curvature R1, R2, R3, R4, R5 for a family of femoral component sizes 1 to 10. As shown in the table 2000, the length of each radius of curvature R1, R2, R3, R4, R5 for each size 1 to 10 of the femoral component 1700 is selected such that the ratios of radii of curvature fall within the predetermined boundaries. In some construction, some or all of the ratios of radii may be maintained at a substantial constant ratio value across the femoral sizes 1 to 10.

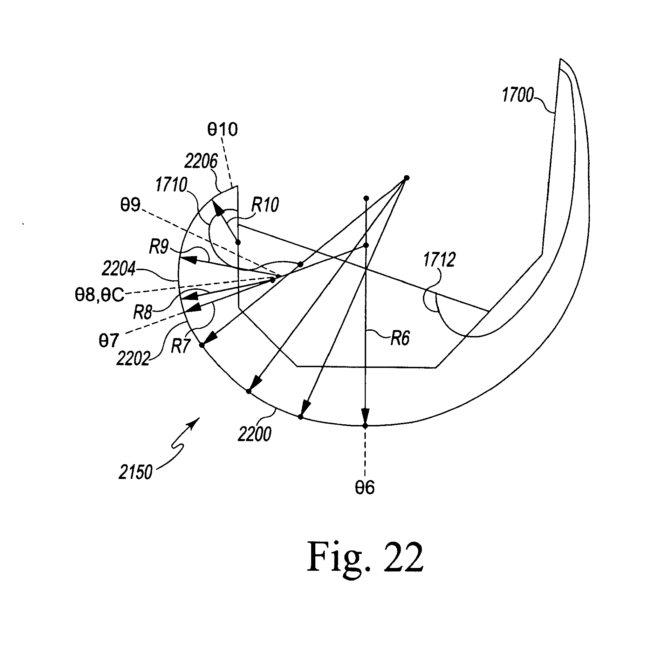

The overall shape and design of the condyle surface 1720 of the femoral component 1700 has been described above in relation to a single condyle 1704, 1706 of the femoral component 1700. In some constructions, both condyles 1704, 1706 of the femoral component 1700 may be symmetrical and have similar condyle surfaces 1720. However, the condyles 1704, 1706 of the femoral component 1700 may be asymmetrical. For example, as shown in FIG. 22, the femoral component 1700 may include a second condyle 1704, 1706 having a condyle surface 2150, which is defined in part by a plurality of curved surface sections 2200, 2202, 2204, 2206. The curved surface section 2200 extends from an earlier degree of flexion θ6 to a later degree of flexion θ7. The curved surface section 2202 extends from the degree of flexion θ7 to a later degree of flexion θ8. The curved surface section 2204 extends from the degree of flexion θ8 to a later degree of flexion θ9. The curved surface section 2206 extends from the degree of flexion θ9 to a later degree of flexion θ10. The condyle surface 2150 also includes a distal radius R6, which is gradually transitioned to a radius of curvature R7 via the curved surface section 2200. Additionally, the curved surface section 2202 is defined by a radius of curvature R8, the curved surface section 2204 is defined by a radius of curvature R9, and the curved surface section 2206 is defined by a radius of curvature R10.

As such, when the condyles 1704, 1706 are symmetrical, the degree of flexion θ6 is substantially equal to the degree of flexion θ1, the degree of flexion θ7 is substantially equal to the degree of flexion θ2, the degree of flexion θ8 is substantially equal to the degree of flexion θ3, the degree of flexion θ9 is substantially equal to the degree of flexion θ4, and the degree of flexion θ10 is substantially equal to the degree of flexion θ5. Additionally, the radius of curvature R6 is substantially equal to the radius of curvature R1, the radius of curvature R7 is substantially equal to the radius of curvature R2, the radius of curvature R8 is substantially equal to the radius of curvature R3, the radius of curvature R9 is substantially equal to the radius of curvature R4, and the radius of curvature R10 is substantially equal to the radius of curvature R5. Further, the set of coefficient values "a", b", "c", and/or "d" of the equation (4) described above are substantially similar for both condyles.

However, in other constructions, the condyles 1704, 1706 are asymmetrical. As such, the degree of flexion θ6 may be different from the degree of flexion θ1. Additionally, the degree of flexion θ7 may be different from the degree of flexion θ2, the degree of flexion θ8 may be different from the degree of flexion θ3, the degree of flexion θ9 may be different from the degree of flexion θ4, and/or the degree of flexion θ10 may be different from the degree of flexion θ5. Additionally, when the condyles 1704, 1706 are asymmetrical, the radius of curvature R6 may be different from the radius of curvature R1, the radius of curvature R7 may be different from the radius of curvature R2, the radius of curvature R8 may be different from the radius of curvature R3, the radius of curvature R9 may be different from the radius of curvature R4, and/or the radius of curvature R10 may be different from the radius of curvature R5. Further, the set of coefficient values "a", b", "c", and/or "d" of the equation (3) described above may be different between the condyle surfaces 1720 and 2150.

## Claims

1. A posterior stabilized orthopaedic knee prosthesis comprising:

   a femoral component (1700) including (i) a pair of spaced apart condyles (1704, 1706) defining an intercondylar notch (1708) therebetween, at least one of the pair of spaced apart condyles having a condyle surface (1720) curved in the sagittal plane and (ii) a posterior cam (1710) positioned in the intercondylar notch, and
   a tibial bearing (14) including (i) a platform (30) having a bearing surface (32) configured to articulate with the condyle surface of the femoral component and (ii) a spine (60) extending upwardly from the platform,
   in which the condyle surface of the femoral component (i) contacts the bearing surface at a first contact point (1852) on the condyle surface at a first degree of flexion (θ1), (ii) contacts the bearing surface at a second contact point (1852) on the condyle surface at a second degree of flexion (θ2), the second degree of flexion being greater than the first degree of flexion and in the range of 0 degrees to 75 degrees, and (iii) contacts the bearing surface at a third contact point (1852) on the condyle surface at a third degree of flexion (θ3), the third degree of flexion being greater than the second degree of flexion and less than about 90 degrees,
   in which the condyle surface has a first radius of curvature (R1) in the sagittal plane at the first contact point, a second radius of curvature (R2) at the second contact point, and a third radius of curvature (R3) at the third contact point,
   **<u>characterised in that</u>** the condyle surface has a first curved surface section (1802) defined between the first contact point and the second contact point, the first curved surface section having a non-constant radius of curvature, and the posterior cam of the femoral component initially contacts the spine of the tibial bearing at a degree of flexion (θC) between the second degree of flexion and the third degree of flexion.

2. The posterior stabilized orthopaedic knee prosthesis of claim 1, in which the posterior cam (1710) of the femoral component (1700) includes a concave cam surface (1716) and a convex cam surface (1718) that are positioned toward a posterior side of the femoral component.

3. The posterior stabilized orthopaedic knee prosthesis of claim 1, in which the first degree of flexion is about 0 degrees and the second degree of flexion is about 70 degrees.

4. The posterior stabilized orthopaedic knee prosthesis of claim 3, in which the third degree of flexion is no less than 73 degrees.

5. The posterior stabilized orthopaedic knee prosthesis of claim 4, the femoral component (1700) initially contacts the spine (60) of the tibial bearing (14) at a degree of flexion in the range of 70 degrees to 80 degrees.

6. The posterior stabilized orthopaedic knee prosthesis of claim 1, in which (i) the condyle surface (1720) of the femoral component (1700) contacts the bearing surface (32) at a fourth contact point (1852) on the condyle surface at a fourth degree of flexion (θ4), the fourth degree of flexion being greater than the third degree of flexion (θ3) and (ii) has a fourth radius of curvature (R4) in the sagittal plane at the fourth contact point, in which the ratio of the fourth radius of curvature (R4 to the third radius of curvature (R3) is in the range of 0.7 to 1.15.

7. The posterior stabilized orthopaedic knee prosthesis of claim 6, in which the fourth degree of flexion is in the range of 90 degrees to 120 degrees.

8. The posterior stabilized orthopaedic knee prosthesis of claim 6, in which (i) the condyle surface of the femoral component contacts the bearing surface at a fifth contact point (1852) on the condyle surface at a fifth degree of flexion (θ5), the fifth degree of flexion being greater than the fourth degree of flexion (θ4) and (ii) has a fifth radius (R5) of curvature in the sagittal plane at the fifth contact point, in which the fifth radius of curvature is less than the fourth radius of curvature (R4).

9. The posterior stabilized orthopaedic knee prosthesis of claim 7, in which the fifth degree of flexion (θ5) is in the range of 140 degrees to 165 degrees.

10. The posterior stabilized orthopaedic knee prosthesis of claim 1, in which (i) the first radius of curvature (R1) is greater than the second radius of curvature (R2) and (ii) the first curved surface section (1802)has an anterior-posterior decreasing, non-constant radius of curvature.

11. The posterior stabilized orthopaedic knee prosthesis of claim 10, in which the third radius of curvature (R3) is no greater than the second radius of curvature (R2).

## Patentansprüche

1. Posterior stabilisierte orthopädische Knieprothese, umfassend:

eine Femurkomponente (1700) mit (i) einem Paar beabstandeter Kondylen (1704, 1706), die einen interkondylären Notch (1708) zwischen einander begrenzen, wobei zumindest eine des Paars beabstandeter Kondylen eine Kondylenoberfläche (1720) aufweist, die in der Sagittalebene gekrümmt ist, und (ii) einem posterioren Nocken (1710), der in dem interkondylären Notch angeordnet ist; und
ein Tibialager (14) mit (i) einer Platte (30), die eine Lagerfläche (32) aufweist, welche dafür konfiguriert ist, mit der Kondylenoberfläche der Femurkomponente ein Gelenk zu bilden, und (ii) einem Dorn (60), welcher von der Platte aus nach oben verläuft,
wobei die Kondylenoberfläche der Femurkomponente (i) die Lagerfläche an einem ersten Kontaktpunkt (1825) auf der Kondylenoberfläche bei einem ersten Beugungsgrad (θ1) berührt, (ii) die Lagerfläche an einem zweiten Kontaktpunkt (1825) auf der Kondylenoberfläche bei einem zweiten Beugungsgrad (θ2) berührt, wobei der zweite Beugungsgrad größer als der erste Beugungsgrad ist und in einem Bereich von 0 bis 75 Grad liegt, und (iii) die Lagerfläche an einem dritten Kontaktpunkt (1825) auf der Kondylenoberfläche bei einem dritten Beugungsgrad (θ3) berührt, wobei der dritte Begungsgrad größer als der zweite Beugungsgrad ist und unter ungefähr 90 Grad liegt,
wobei die Kondylenoberfläche einen ersten Krümmungsradius (R1) in der Sagittalebene an dem ersten Kontaktpunkt, einen zweiten Krümmungsradius (R2) an dem zweiten Kontaktpunkt und einen dritten Krümmungsradius (R3) an dem dritten Kontaktpunkt hat,
**dadurch gekennzeichnet, dass** die Kondylenoberfläche einen zwischen dem ersten Kontaktpunkt und dem zweiten Kontaktpunkt gebildeten ersten gekrümmten Oberflächenabschnitt (1802) hat, wobei der erste gekrümmte Oberflächenabschnitt einen nicht konstanten Krümmungsradius hat, und dass der posteriore Nocken der Femurkomponente anfangs den Dorn des Tibialagers bei einem Beugungsgrad (θC) zwischen dem zweiten Beugungsgrad und dem dritten Beugungsgrad berührt.

2. Posterior stabilisierte orthopädische Knieprothese nach Anspruch 1, wobei der posteriore Nocken (1710) der Femurkomponente (1700) eine konkave Nockenfläche (1716) und eine konvexe Nockenfläche (1718) hat, welche zu einer posterioren Seite der Femurkomponente hin positioniert sind.

3. Posterior stabilisierte orthopädische Knieprothese nach Anspruch 1, wobei der erste Begungsgrad ungefähr 0 Grad ist und der zweite Begungsgrad ungefähr 70 Grad ist.

4. Posterior stabilisierte orthopädische Knieprothese nach Anspruch 3, wobei der dritte Beugungsgrad nicht unter 73 Grad liegt.

5. Posterior stabilisierte orthopädische Knieprothese nach Anspruch 4, wobei die Femurkomponente (1700) den Dorn (60) des Tibialagers (14) anfangs bei einem Beugungsgrad von 70 bis 80 Grad berührt.

6. Posterior stabilisierte orthopädische Knieprothese nach Anspruch 1, wobei (i) die Kondylenoberfläche (1720) der Femurkomponente (1700) die Lagerfläche (32) an einem vierten Kontaktpunkt (1852) auf der Kondylenoberfläche bei einem vierten Beugungsgrad ($\theta$4) berührt, wobei der vierte Beugungsgrad größer als der dritte Beugungsgrad ($\theta$3) ist, und (ii) einen vierten Krümmungsradius (R4) in der Sagittalebene an dem vierten Kontaktpunkt hat, wobei das Verhältnis von viertem Krümmungsradius zu drittem Krümmungsradius im Bereich von 0,7 bis 1,15 liegt.

7. Posterior stabilisierte orthopädische Knieprothese nach Anspruch 6, wobei der vierte Beugungsgrad im Bereich von 90 Grad bis 120 Grad liegt.

8. Posterior stabilisierte orthopädische Knieprothese nach Anspruch 6, wobei (i) die Kondylenoberfläche der Femurkomponente die Lagerfläche an einem fünften Kontaktpunkt (1852) auf der Kondylenoberfläche bei einem fünften Beugungsgrad ($\theta$5) berührt, wobei der fünfte Beugungsgrad größer als der vierte Beugungsgrad ($\theta$4) ist, und (ii) einen fünften Krümmungsradius (R5) in der Sagittalebene an dem fünften Kontaktpunkt hat, wobei der fünfte Krümmungsradius kleiner als der vierte Krümmungsradius (R4) ist.

9. Posterior stabilisierte orthopädische Knieprothese nach Anspruch 7, wobei der fünfte Beugungsgrad ($\theta$5) im Bereich von 140 Grad bis 165 Grad liegt.

10. Posterior stabilisierte orthopädische Knieprothese nach Anspruch 1, wobei (i) der erste Krümmungsradius (R1) größer als der zweite Krümmungsradius (R2) ist und (ii) der erste gekrümmte Oberflächenabschnitt (1802) einen anterior-posterior abnehmenden, nicht konstanten Krümmungsradius hat.

11. Posterior stabilisierte orthopädische Knieprothese nach Anspruch 10, wobei der dritte Krümmungsradius (R3) nicht größer als der zweite Krümmungsradius (R2) ist.

**Revendications**

1. Prothèse orthopédique de genou stabilisée postérieure comprenant :

    un composant fémoral (1700) comprenant (i) une paire de condyles espacés (1704, 1706) définissant une encoche intercondylienne (1708) entre eux, au moins l'un de la paire de condyles espacés ayant une surface condylienne (1720) incurvée dans le plan sagittal et (ii) une came postérieure (1710) positionnée dans l'encoche intercondylienne, et
    un appui tibial (14) comprenant (i) une plateforme (30) ayant une surface d'appui (32) configurée pour s'articuler avec la surface condylienne du composant fémoral et (ii) une épine (60) s'étendant vers le haut à partir de la plateforme,
    dans laquelle la surface condylienne du composant fémoral (i) est en contact avec la surface d'appui au niveau d'un premier point de contact (1852) sur la surface condylienne à un premier degré de flexion ($\theta$1), (ii) est en contact avec la surface d'appui au niveau d'un deuxième point de contact (1852) sur la surface condylienne à un deuxième degré de flexion ($\theta$2), le deuxième degré de flexion étant supérieur au premier degré de flexion et dans la plage de 0 degré à 75 degrés, et (iii) est en contact avec la surface d'appui au niveau d'un troisième point de contact (1852) sur la surface condylienne à un troisième degré de flexion ($\theta$3), le troisième degré de flexion étant supérieur au deuxième degré de flexion et inférieur à environ 90 degrés,
    dans laquelle la surface condylienne a un premier rayon de courbure (R1) dans le plan sagittal au niveau d'un premier point de contact, un deuxième rayon de courbure (R2) au niveau du deuxième point de contact et un troisième rayon de courbure (R3) au niveau du troisième point de contact,
    **caractérisée en ce que** la surface condylienne a une première section de surface incurvée (1802) définie entre le premier point de contact et le deuxième point de contact, la première section de surface incurvée ayant un rayon de courbure non constant, et la came postérieure du composant fémoral est initialement en contact avec l'épine de l'appui tibial à un degré de flexion ($\theta$C) entre le deuxième degré de flexion et le troisième degré de flexion.

2. Prothèse orthopédique de genou stabilisée postérieure selon la revendication 1, dans laquelle la came postérieure (1710) du composant fémoral (1700) comprend une surface de came concave (1716) et une surface de came convexe (1718) qui sont positionnées vers un côté postérieur du composant fémoral.

3. Prothèse orthopédique de genou stabilisée postérieure selon la revendication 1, dans laquelle le premier degré de flexion est d'environ de 0 degré et le deuxième degré de flexion est d'environ 70 degrés.

4. Prothèse orthopédique de genou stabilisée postérieure selon la revendication 3, dans laquelle le troisième degré de flexion n'est pas inférieur à 73 degrés.

5. Prothèse orthopédique de genou stabilisée postérieure selon la revendication 4, le composant fémoral (1700) est initialement en contact avec l'épine (60) de l'appui tibial (14) à un degré de flexion dans la plage de 70 degrés à 80 degrés.

6. Prothèse orthopédique de genou stabilisée postérieure selon la revendication 1, dans laquelle (i) la surface condylienne (1720) du composant fémoral (1700) est en contact avec la surface d'appui (32) à un quatrième point de contact (1852) sur la surface condylienne à un quatrième degré de flexion ($\theta$4), le quatrième degré de flexion étant supérieur au troisième degré de flexion ($\theta$3) et (ii) a un quatrième rayon, de courbure (R4) dans le plan sagittal au niveau du quatrième point de contact, dans laquelle le rapport du quatrième rayon de courbure (R4) sur le troisième rayon de courbure (R3) est de l'ordre de 0,7 à 1,15.

7. Prothèse orthopédique de genou stabilisée postérieure selon la revendication 6, dans laquelle le quatrième degré de flexion est dans la plage de 90 degrés à 120 degrés.

8. Prothèse orthopédique de genou stabilisée postérieure selon la revendication 6, dans laquelle (i) la surface condylienne du composant fémoral est en contact avec la surface d'appui au niveau d'un cinquième point de contact (1852) sur la surface condylienne à un cinquième degré de flexion ($\theta$5), le cinquième degré de flexion étant supérieur au quatrième degré de flexion ($\theta$4) et (ii) a un cinquième rayon (R5) de courbure dans le plan sagittal au niveau du cinquième point de contact, dans laquelle le cinquième rayon de courbure est inférieur au quatrième rayon de courbure (R4).

9. Prothèse orthopédique de genou stabilisée postérieure selon la revendication 7, dans laquelle le cinquième degré de flexion ($\theta$5) est dans la plage de 140 degrés à 165 degrés.

10. Prothèse orthopédique de genou stabilisée postérieure selon la revendication 1, dans laquelle (i) le premier rayon de courbure (R1) est supérieur au deuxième rayon de courbure (R2) et (ii) la première section de surface incurvée (1802) a un rayon de courbure non constant, décroissant de l'avant vers l'arrière.

11. Prothèse orthopédique de genou stabilisée postérieure selon la revendication 10, dans laquelle le troisième rayon de courbure (R3) n'est pas supérieur au deuxième rayon de courbure (R2).

Fig. 1

Fig. 2

Fig. 3

Fig. 4

Fig. 5

Fig. 6

Fig. 7

# Fig. 8

0.5mm RADIUS INCREASE AT 30,50,70 AND 90 deg

200

AP TRANSLATION OF CLP (mm)

FLEXION (deg)

Legend:
- constant med
- constant lat
- 30 deg med
- 30 deg lat
- 50 deg lat
- 50 deg med
- 70 deg med
- 70 deg lat
- 90 deg med
- 90 deg lat

Fig. 9

EP 2 726 020 B1

**1 mm RADIUS INCREASE AT 30,50,70 AND 90 deg**

*300*

AP TRANSLATION
OF CLP (mm)

Legend:
- constant med
- constant lat
- 30 deg med
- 30 deg lat
- 50 deg lat
- 50 deg med
- 70 deg med
- 70 deg lat
- 90 deg med
- 90 deg lat

FLEXION (deg)

**Fig. 10**

Fig. 11

Fig. 12

Fig. 13

| Component Size | Origin Distance | RAY LENGTH EQUATION |
|---|---|---|
| 1 | 4.008 | $R=29.383391+0.0166694187*\theta-0.00027002378*\theta^2-0.0000124837*\theta^3$ |
| 2 | 3.898 | $R=30.470577+0.0166694187*\theta-0.00027002378*\theta^2-0.0000124837*\theta^3$ |
| 3 | 3.722 | $R=31.597988+0.0166694187*\theta-0.00027002378*\theta^2-0.0000124837*\theta^3$ |
| 4 | 3.629 | $R=32.767114+0.0166694187*\theta-0.00027002378*\theta^2-0.0000124837*\theta^3$ |
| 5 | 3.468 | $R=33.979497+0.0166694187*\theta-0.00027002378*\theta^2-0.0000124837*\theta^3$ |
| 6 | 3.288 | $R=35.236738+0.0166694187*\theta-0.00027002378*\theta^2-0.0000124837*\theta^3$ |
| 7 | 3.088 | $R=36.540498+0.0166694187*\theta-0.00027002378*\theta^2-0.0000124837*\theta^3$ |
| 8 | 2.866 | $R=37.892496+0.0166694187*\theta-0.00027002378*\theta^2-0.0000124837*\theta^3$ |
| 9 | 2.623 | $R=39.294518+0.0166694187*\theta-0.00027002378*\theta^2-0.0000124837*\theta^3$ |
| 10 | 2.356 | $R=40.748416+0.0166694187*\theta-0.00027002378*\theta^2-0.0000124837*\theta^3$ |

700

## Fig. 14

EP 2 726 020 B1

| Component Size | R1 | R2 | R3 | R4 | R5 | Ratio R1/R2 | Ratio R1/R3 | Ratio R1/R4 |
|---|---|---|---|---|---|---|---|---|
| 1 | 25.5 | 20.4 | 25.4 | 11.2 | 8.1 | 1.251 | 1.005 | 2.271 |
| 2 | 26.7 | 21.3 | 26.6 | 12.6 | 7.9 | 1.252 | 1.005 | 2.120 |
| 3 | 28.0 | 22.3 | 27.8 | 14.0 | 7.7 | 1.253 | 1.005 | 2.001 |
| 4 | 29.3 | 23.3 | 29.1 | 15.4 | 7.5 | 1.255 | 1.005 | 1.901 |
| 5 | 30.7 | 24.4 | 30.5 | 16.9 | 7.3 | 1.257 | 1.005 | 1.818 |
| 6 | 32.1 | 25.5 | 31.9 | 18.4 | 7.1 | 1.259 | 1.005 | 1.747 |
| 7 | 33.6 | 26.7 | 33.4 | 19.9 | 6.8 | 1.261 | 1.005 | 1.686 |
| 8 | 35.2 | 27.9 | 35.0 | 21.5 | 6.6 | 1.263 | 1.005 | 1.633 |
| 9 | 36.8 | 29.1 | 36.7 | 23.2 | 6.3 | 1.265 | 1.005 | 1.586 |
| 10 | 38.6 | 30.4 | 38.4 | 25.0 | 6.1 | 1.268 | 1.005 | 1.545 |

800

# Fig. 15

Fig. 16

Fig. 17

Fig. 18

*1900*

| Component Size | Origin Distance | Ray Length Equation |
|---|---|---|
| 1 | 5.092 | $R = 33.289093 + 0.0153846150*\theta - 0.0002702378*\theta^2 - 0.0000212*\theta^3$ |
| 2 | 4.928 | $R = 34.454211 + 0.0153846150*\theta - 0.0002702378*\theta^2 - 0.0000212*\theta^3$ |
| 3 | 4.743 | $R = 35.660109 + 0.0153846150*\theta - 0.0002702378*\theta^2 - 0.0000212*\theta^3$ |
| 4 | 4.534 | $R = 36.908213 + 0.0153846150*\theta - 0.0002702378*\theta^2 - 0.0000212*\theta^3$ |
| 5 | 4.301 | $R = 38.200000 + 0.0153846150*\theta - 0.0002702378*\theta^2 - 0.0000212*\theta^3$ |
| 6 | 3.891 | $R = 39.384200 + 0.0153846150*\theta - 0.0002702378*\theta^2 - 0.0000212*\theta^3$ |
| 7 | 3.444 | $R = 40.605110 + 0.0153846150*\theta - 0.0002702378*\theta^2 - 0.0000212*\theta^3$ |
| 8 | 3.199 | $R = 42.107972 + 0.0153846150*\theta - 0.0002702378*\theta^2 - 0.0000212*\theta^3$ |
| 9 | 2.763 | $R = 43.497535 + 0.0153846150*\theta - 0.0002702378*\theta^2 - 0.0000212*\theta^3$ |
| 10 | 2.290 | $R = 44.932954 + 0.0153846150*\theta - 0.0002702378*\theta^2 - 0.0000212*\theta^3$ |

Fig. 19

| Component Size | R1 | R2 | R3 | R4 | R5 | R2/R1 | R3/R2 | R4/R3 | R5/R4 |
|---|---|---|---|---|---|---|---|---|---|
| 1 | 28.3010 | 18.2900 | 13.7340 | 15.6900 | 10.8180 | 0.6463 | 0.7509 | 1.1424 | 0.6895 |
| 2 | 29.6310 | 19.1350 | 14.3800 | 16.3020 | 11.3270 | 0.6458 | 0.7515 | 1.1337 | 0.6948 |
| 3 | 31.0230 | 20.0180 | 15.0560 | 16.7660 | 11.8590 | 0.6453 | 0.7521 | 1.1136 | 0.7073 |
| 4 | 32.4810 | 20.9390 | 15.7630 | 16.8070 | 12.4160 | 0.6446 | 0.7528 | 1.0662 | 0.7388 |
| 5 | 34.0080 | 21.9000 | 16.5040 | 15.5000 | 13.0000 | 0.6440 | 0.7536 | 0.9392 | 0.8387 |
| 6 | 35.6060 | 22.7870 | 19.3370 | 16.2290 | 13.6110 | 0.6400 | 0.8486 | 0.8393 | 0.8387 |
| 7 | 37.2800 | 23.7080 | 22.4110 | 16.9910 | 14.2510 | 0.6359 | 0.9453 | 0.7582 | 0.8387 |
| 8 | 39.0320 | 24.8500 | 23.1700 | 18.1470 | 14.9210 | 0.6367 | 0.9324 | 0.7832 | 0.8222 |
| 9 | 40.8670 | 25.9150 | 24.2500 | 20.6510 | 15.6220 | 0.6341 | 0.9358 | 0.8516 | 0.7565 |
| 10 | 42.7870 | 27.0220 | 25.5480 | 25.5740 | 16.3560 | 0.6315 | 0.9455 | 0.9971 | 0.6420 |

2000

# Fig. 20

Fig. 21

Fig. 22

EP 2 726 020 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20090326665 A
- US 20090326666 A
- US 20090326667 A